# EUROPÄISCHE PATENTSCHRIFT

(11) **EP 3 584 005 B1**
(45) Veröffentlichungstag und Bekanntmachung des Hinweises auf die Patenterteilung: **11.11.2020**
(21) Anmeldenummer: 19180576.1
(22) Anmeldetag: 17.06.2019
(51) Int. Cl.: B01F 11/00, B01F 13/00, B01F 15/00, B01F 15/02, A61B 17/88

(54) **KNOCHENZEMENTAPPLIKATOR MIT VERSENKBAREM MISCHSTAB UND VERFAHREN ZUR HERSTELLUNG EINES KNOCHENZEMENTS**
BONE CEMENT APPLICATOR WITH RETRACTABLE MIXING ROD AND METHOD FOR PRODUCING A BONE CEMENT
APPLICATEUR DE CIMENT OSSEUX POURVU DE BÂTON MÉLANGEUR POUVANT ÊTRE ABAISSÉ ET PROCÉDÉ DE FABRICATION D'UN CIMENT OSSEUX

(30) Priorität: 18.06.2018 DE 102018209807
(43) Veröffentlichungstag der Anmeldung: 25.12.2019
(73) Patentinhaber: Heraeus Medical GmbH, 61273 Wehrheim (DE)
(72) Erfinder: Vogt, Sebastian, 61273 Wehrheim (DE); Kluge, Thomas, 61273 Wehrheim (DE)
(74) Vertreter: Heraeus IP

(56) Entgegenhaltungen:
- WO-A1-97/18031
- DE-A1-102016 121 607

## Beschreibung

Die Erfindung betrifft einen Knochenzementapplikator zum Lagern und Mischen eines Knochenzementpulvers und einer Monomerflüssigkeit sowie zum Applizieren eines aus dem Knochenzementpulver und der Monomerflüssigkeit gemischten pastösen Knochenzements. Die Erfindung betrifft auch ein Verfahren zur Herstellung eines Knochenzements mit einem solchen Knochenzementapplikator.

Gegenstand der Erfindung ist ein Knochenzementapplikator zum Lagern, Mischen und Applizieren von Knochenzement. Die Knochenzementapplikator wird vorzugsweise durch ein geschlossenes Prepack-Mischsystem mit einer integrierten Auspressvorrichtung realisiert. Der Knochenzementapplikator ist vorzugsweise für die Arthoplasty, die Vertebroplasty und die Kyphoplasty geeignet beziehungsweise bestimmt. Es werden hierzu auch Verfahren zum Mischen und Applizieren von Polymethylmethacrylat-Knochenzement vorgeschlagen.

Polymethylmethacrylat-(PMMA)-Knochenzemente gehen auf die grundlegenden Arbeiten von Sir Charnley zurück (Charnley, J.: Anchorage of the femoral head prosthesis of the shaft of the femur. J. Bone Joint Surg. 42 (1960) 28-30). Konventionelle PMMA-Knochenzemente bestehen aus einer flüssigen Monomerkomponente und einer Pulverkomponente. Die Monomerkomponente enthält im Allgemeinen das Monomer Methylmethacrylat und einen darin gelösten Aktivator (N,N-Dimethyl-p-toluidin). Die Pulverkomponente, auch als Knochenzementpulver bezeichnet, weist ein oder mehrere Polymere auf, die auf Basis von Methylmethacrylat und Comonomeren, wie Styren, Methylacrylat oder ähnlichen Monomeren durch Polymerisation, vorzugsweise Suspensionspolymerisation, hergestellt sind, einen Röntgenopaker und den Initiator Dibenzoylperoxid auf. Beim Vermischen der Pulverkomponente mit der Monomerkomponente entsteht durch Quellung der Polymere der Pulverkomponente im Methylmethacrylat ein plastisch verformbarer Teig, der eigentliche Knochenzement beziehungsweise Knochenzementteig. Beim Vermischen der Pulverkomponente mit der Monomerkomponente reagiert der Aktivator N,N-Dimethyl-p-toluidin mit Dibenzoylperoxid unter Bildung von Radikalen. Die gebildeten Radikale initiieren die radikalische Polymerisation des Methylmethacrylats. Mit fortschreitender Polymerisation des Methylmethacrylats erhöht sich die Viskosität des Knochenzementteigs, bis dieser erstarrt. PMMA-Knochenzemente können in geeigneten Mischbechern mit Hilfe von Spateln durch Vermischen des Knochenzementpulvers mit der Monomerflüssigkeit vermischt werden. Dabei kann es zum Einschluss von Luftblasen im Knochenzementteig kommen, welche die mechanischen Eigenschaften des ausgehärteten Knochenzements negativ beeinflussen können.

Zur Vermeidung von Lufteinschlüssen im Knochenzementteig wurden eine Vielzahl von Vakuum-Zementiersystemen beschrieben, von denen exemplarisch folgende genannt sind: US 6 033 105 A, US 5 624 184 A, US 4 671 263 A, US 4 973 168 A, US 5 100 241 A, WO 99/67015 A1, EP 1 020 167 A2, US 5 586 821 A, EP 1 016 452 A2, DE 36 40 279 A1, WO 94/26403 A1, EP 1 005 901 A2, EP 1 886 647 A1, US 5 344 232 A. Diese Mischsysteme enthalten zur Vermischung der Zementkomponenten einen manuell von außen zu bedienenden Mischstab an dem Mischflügel als Mischer angebracht sind. Zur Erzeugung des Vakuums werden externe Vakuumpumpen benötigt. Diese Vakuumpumpen werden im Allgemeinen mit Druckluft angetrieben und erzeugen Vakuum nach dem Venturiprinzip. Für das Auspressen des gemischten Knochenzements aus den Kartuschen nutzt man manuell angetriebene Auspressvorrichtungen. Diese Auspressvorrichtungen können mit den Kartuschen zum Auspressen des Zementteigs reversibel verbunden werden. Nach dem Auspressvorgang werden die Auspressvorrichtungen von den Kartuschen getrennt, gereinigt und resterilisiert. Die verwendeten Kartuschen werden entsorgt.

Eine Weiterentwicklung in der Zementiertechnik stellen Zementiersysteme dar, in denen sowohl das Knochenzementpulver als auch die Monomerflüssigkeit bereits in separaten Kompartimenten der Mischvorrichtungen verpackt sind und die erst unmittelbar vor der Zementapplikation im Zementiersystem miteinander vermischt werden. Solche geschlossenen Full-Prepacked-Mischvorrichtungen wurden mit der EP 0 692 229 A1, der DE 10 2009 031 178 B3, der US 5 997 544 A, der US 6 709 149 B1, der WO 00/35506 A1, der EP 0 796 653 A2 und der US 5 588 745 A vorgeschlagen.

Das Patent DE 10 2009 031 178 B3 offenbart eine Lager- und Mischvorrichtung als Full-Prepacked-Mischvorrichtung, in dem die zur Herstellung des Knochenzements notwendigen Ausgangskomponenten bereits in der Lager- und Mischvorrichtung gelagert sind und in der Lager- und Mischvorrichtung zusammengeführt und gemischt werden können. Die Lager- und Mischvorrichtung weist einen zweiteiligen Austragskolben zum Verschluss einer Zementkartusche auf. Dabei wird eine Kombination aus einem gasdurchlässigen Sterilisationskolben und einem gasundurchlässigen Dichtungskolben verwendet. Dieses Prinzip eines geschlossenen Vakuummischsystems wird beim dem geschlossenen Zementiersystem PALACOS® PRO verwirklicht, das von der Firma Heraeus Medical GmbH hergestellt und vertrieben wird. Für den Monomertransfer und das Mischen unter Vakuum wird eine externe Vakuumpumpe benötigt, die normalerweise durch Druckluft angetrieben wird. Zum Auspressen des gemischten Zementteigs wird ebenfalls eine separate, manuell zu bedienende Auspressvorrichtung benutzt.

In der DE 10 2016 121 607 A1 wird ein Full-Prepacked-Mischsystem mit einer Kartusche enthaltend ein Knochenzementpulver zur Herstellung eines Knochenzements vorgeschlagen. In der Kartusche ist ein Austragskolben vorgesehen und hinter der Kartusche eine Aufnahme enthaltend einen Monomerflüssigkeitsbehälter angeordnet. An der Rückseite der Aufnahme befindet sich ein Austragskolben, mit dem der Monomerflüssigkeitsbehälter zerquetscht werden kann und die Monomerflüssigkeit aus der Aufnahme in die Kartusche gepresst werden kann. Bei diesem System erfolgt keine manuelle Durchmischung der Ausgangskomponenten mit Hilfe eines Mischers.

In der WO 97/18031 A1 wird eine Vorrichtung zum sukzessiven Zuführen von Chargen in ein Mischgefäß unter Teilvakuum zur Herstellung von Knochenzement beschrieben.

Bei den bisher referenzierten Vakuummischsystemen mit Mischer muss nach der Vermischung der Zementkomponenten, vor der Applikation des Knochenzements, der Mischstab entweder abgebrochen oder aus dem Mischsystem herausgezogen werden. Die bekannten Verfahren und Vorrichtungen haben dadurch den Nachteil, dass es beim Abbrechen zu einer Undichtigkeit des Knochenzementapplikators kommen kann und dass das Abbrechen sowie das Herausziehen des Mischstabs immer als zusätzliche Arbeitsschritte notwendig sind. Zudem liegt der abgebrochene Mischstab als ein weiteres separates Teil im OP-Saal herum, das entsorgt werden muss. Bei Knochenzementapplikatoren ohne Mischer muss einiger Aufwand betrieben werden, damit der Knochenzement ausreichend durchmischt ist. Zudem kann es stets dazu kommen, dass Teile des Knochenzements nicht ausreichend durchmischt sind. Diese müssen entfernt oder zurückgehalten werden oder es kann zu einer Beeinträchtigung der Qualität des Knochenzements kommen.

Die Erfindung hat die Aufgabe, einen Knochenzementapplikator zum Lagern, Mischen und Applizieren von Polymethylmethacrylat-Knochenzement zu entwickeln, mit dem die Nachteile des Stands der Technik überwunden werden. In diesem Knochenzementapplikator sollen das Knochenzementpulver und die Monomerflüssigkeit in separaten Kompartimenten vor ihrer Vermischung gelagert werden. Der Monomertransfer aus dem Monomerflüssigkeitsbehälter in das Knochenzementpulver soll ohne Anlegen eines extern bereitgestellten Vakuums erfolgen. Die Vermischung soll durch einen Mischstab mit einem Mischer in der geschlossenen Vorrichtung derart erfolgen, dass der medizinische Anwender nicht mit dem Knochenzementpulver oder der Monomerflüssigkeit in Berührung kommt. Nach der Vermischung der Zementkomponenten soll eine Entfernung des Mischstabs vom Mischsystem durch Herausziehen und/oder Abbrechen des Mischstabs vermieden werden. Der gebildete Knochenzement soll aus dem Knochenzementapplikator manuell auspressbar sein, ohne dass eine externe Auspressvorrichtung an die Vorrichtung angeschlossen werden muss.

Die Erfindung hat deshalb die Aufgabe, ein komplett autonomes Prepack-Mischystem zu entwickeln, das eine Vermischung der Zementkomponenten und ein Auspressen des gemischten Knochenzements erlaubt, ohne dass Zusatzgeräte wie externe Vakuumpumpen und Auspressvorrichtungen erforderlich sind.

Die Aufgaben der Erfindung werden gelöst durch einen Knochenzementapplikator zum Lagern und Mischen eines Knochenzementpulvers und einer Monomerflüssigkeit sowie zum Applizieren eines aus dem Knochenzementpulver und der Monomerflüssigkeit gemischten pastösen Knochenzements, der Knochenzementapplikator aufweisend
A) eine Kartusche mit einem zylindrischen Innenraum zum Mischen des Knochenzements, wobei die Kartusche an einer Vorderseite einen Kartuschenkopf mit einer Austragsöffnung zum Austreiben des Knochenzements aus dem Innenraum aufweist und wobei die Kartusche an einer der Vorderseite der Kartusche gegenüberliegenden Rückseite ein Gewinde aufweist,
B) einen Austragskolben zum Austreiben des gemischten Knochenzements aus dem Innenraum durch die Austragsöffnung, wobei das Knochenzementpulver zwischen dem Austragskolben und dem Kartuschenkopf in dem Innenraum der Kartusche enthalten ist,
C) ein Gewinderohr, das an der von dem Kartuschenkopf abgewandten Rückseite des Austragskolbens angeordnet ist, wobei das Gewinderohr ein zu dem Gewinde an der Rückseite der Kartusche passendes Gegengewinde aufweist und das Gegengewinde des Gewinderohrs in das Gewinde an der Rückseite der Kartusche greift, wobei sich das Gewinderohr an der Rückseite der Kartusche aus der Kartusche heraus erstreckt und wobei der Austragskolben durch Schrauben des Gewinderohrs in oder auf die Kartusche im Innenraum der Kartusche entlang der Zylinderachse des Innenraums in Richtung des Kartuschenkopfs beweglich angeordnet ist,
D) eine Aufnahme, wobei ein Monomerflüssigkeitsbehälter im Inneren der Aufnahme angeordnet ist, wobei der Monomerflüssigkeitsbehälter die Monomerflüssigkeit enthält und im Inneren der Aufnahme zu öffnen ist, wobei die Aufnahme an einer dem Austragskolben gegenüberliegenden Rückseite des Gewinderohrs in dem Gewinderohr steckt und in dem Gewinderohr beweglich ist, und
E) einen Mischstab, wobei der Mischstab mit einem daran befestigten Mischer in dem Innenraum der Kartusche angeordnet ist, wobei der Mischer an einer dem Kartuschenkopf zugewandten Vorderseite des Mischstabs befestigt ist, wobei der Mischstab an einer dem Mischer gegenüberliegenden Seite mit einer dem Austragskolben zugewandten Vorderseite der Aufnahme verbunden ist, wobei der Mischstab durch eine Durchführung in dem Austragskolben geführt ist und in der Durchführung axial beweglich gelagert ist, so dass der Mischstab mit dem Mischer in dem Innenraum zum Durchmischen des Knochenzementpulvers mit der Monomerflüssigkeit durch eine Bewegung der Aufnahme gegen die Kartusche beweglich ist, und wobei der Mischstab lösbar mit der Aufnahme verbunden ist und der von der Aufnahme gelöste Mischstab bei einem Vortreiben der Aufnahme in Richtung des Kartuschenkopfs in die Aufnahme hinein drückbar ist.

Bevorzugt ist die Aufnahme in dem Gewinderohr zumindest axial in Richtung der Zylinderachse des Innenraums der Kartusche beweglich ist. Dabei ist die Aufnahme besonders bevorzugt zumindest bereichsweise in das Gewinderohr einschiebbar.

Die Aufnahme ist vorzugsweise ein Ampullenhalter.

Es kann vorgesehen sein, dass sich ein Teil der Aufnahme, insbesondere ein dem Kartuschenkopf zugewandter Verschluss der Aufnahme, mit dem Mischstab von der restlichen Aufnahme löst.

Der Innenraum der Kartusche hat eine zylindrische Geometrie mit kreisförmiger Grundfläche. Die zylindrische Form ist die einfachste, mit der sich der Innenraum der Kartusche realisieren lässt. Unter einer zylindrischen Form ist geometrisch die Form eines allgemeinen Zylinders mit einer beliebigen Grundfläche zu verstehen, also nicht nur ein Zylinder mit einer kreisförmigen Grundfläche. Der Innenraum der Kartusche benötigt aber eine rotationssymmetrische Symmetrie, das heißt eine zylindrische Form mit kreisförmiger Grundfläche, da ansonsten das Einschrauben des Gewinderohrs nicht möglich ist oder der Austragskolben nicht gut gegen die Innenwand des Innenraums abgedichtet werden könnte. Der zylindrische Raum des Gewinderohrs kann aber durch einen Zylindermantel eines Zylinders mit beliebiger Grundfläche realisiert sein, das heißt auch mit nicht kreisförmigen oder nicht runden Grundflächen, da in den Raum die Aufnahme lediglich eingeschoben werden soll. Erfindungsgemäß wird jedoch eine zylindrische Geometrie mit rotationssymmetrischer und insbesondere kreisrunder Grundfläche auch für den Raum des Gewinderohrs bevorzugt, da diese am einfachsten zu fertigen ist. Das gleiche gilt für das vorzugsweise ebenfalls zylindrisch geformte Innere der Aufnahme.

Bevorzugt ist der Monomerflüssigkeitsbehälter eine Ampulle aus Glas oder aus einem Kunststoff. Ampullen aus Glas oder Kunststoff sind besonders zuverlässig zu öffnen. Zudem kann die Monomerflüssigkeit in solchen Ampullen als Monomerflüssigkeitsbehälter besonders lange gelagert werden. Alternative Monomerflüssigkeitsbehälter könnten beispielsweise beschichtete Folienbeutel sein.

Die Aufnahme ist vorzugsweise als Ampullenhalter ausgebildet. Der Ampullenhalter ist dabei besonders bevorzugt zur Halterung einer Ampulle aus Glas oder Kunststoff geeignet und vorgesehen.

Es kann bevorzugt vorgesehen sein, dass die Austragsöffnung für das Lagern und Mischen mit einem zu öffnenden Verschluss verschlossen ist. Hierdurch wird ein geschlossenes Prepack-Mischsystem bereitgestellt.

Dabei kann vorgesehen sein, dass der Verschluss über ein Gewinde oder einen Bajonett-Verschluss mit dem Kartuschenkopf lösbar verbunden ist.

Es kann vorgesehen sein, dass der Verschluss die Austragsöffnung flüssigkeitsdicht oder gasdicht und flüssigkeitsdicht verschließt.

Hiermit wird sichergestellt, dass beim Mischen des Knochenzements kein Knochenzementpulver, keine Monomerflüssigkeit und kein Knochenzement aus dem Innenraum der Kartusche austreten können.

Alle Teile des Knochenzementapplikators bis auf die Ausgangskomponenten, den Monomerflüssigkeitsbehälter und gegebenenfalls vorhandene Dichtungen bestehen erfindungsgemäß bevorzugt aus Kunststoff, insbesondere einem thermoplastischen Kunststoff. Der Monomerflüssigkeitsbehälter muss, wenn er aus einem Kunststoff besteht, aus einem spröden brechbaren Kunststoff bestehen. Die Dichtungen bestehen vorzugsweise aus Silikon oder Gummi.

Es kann vorgesehen sein, dass das Gewinde an der Rückseite der Kartusche ein Innengewinde ist und das Gegengewinde am Gewinderohr ein Außengewinde ist, so dass der Austragskolben durch Einschrauben des Gewinderohrs in den Innenraum der Kartusche im Innenraum der Kartusche axial entlang der Zylinderachse des Innenraums in Richtung des Kartuschenkopfs bewegbar ist.

Alternativ kann das Gewinde an der Rückseite der Kartusche aber auch ein Außengewinde sein und das Gewinderohr hat ein Innengewinde und wird nach Art einer Hülse auf die Kartusche geschraubt. Das Gewinderohr kann dazu mit einem äußeren Rohr mit dem Innengewinde und mit einem inneren Rohr aufgebaut werden, wobei sich nur das innere Rohr des Gewinderohrs in das Innere der Kartusche erstreckt. Das innere Rohr benötigt dann kein Gewinde. Das Gewinderohr ist dann ein doppelwandiges Rohr mit einem Außengewinde, wobei die beiden Rohrwände an einer dem Kartuschenkopf gegenüberliegenden Rückseite miteinander verbunden sind.

Wenn das Gewinde an der Rückseite der Kartusche ein Innengewinde ist und das Gegengewinde am Gewinderohr ein Außengewinde ist, kann der Knochenzementapplikator besonders kompakt und einfach aufgebaut werden. Das Gewinderohr mit Außengewinde ist dann ein einfaches Rohr, während es mit Innengewinde zweiteilig aufgebaut werden muss, nämlich mit einem äußeren Rohr und innen mit Streben oder einem weiteren Rohr, mit dem der Austragskolben angetrieben wird und in dem die Aufnahme axial beweglich geführt wird.

Des Weiteren kann vorgesehen sein, dass das Gewinderohr einen zylindrischen Raum aufweist, der an der Rückseite des Gewinderohrs offen ist, wobei die Aufnahme von der Rückseite des Gewinderohrs aus in dem zylindrischen Raum des Gewinderohrs steckt.

Die Aufnahme kann so im zylindrischen Raum des Gewinderohrs geführt werden. Zudem kann die Monomerflüssigkeit aus der Aufnahme in den zylindrischen Raum und von dort in den Innenraum der Kartusche geleitet werden beziehungsweise fließen, wobei der zylindrische Raum an seiner Rückseite durch die in den zylindrischen Raum eingesteckte Aufnahme verschlossen ist.

Dabei kann vorgesehen sein, dass die Aufnahme gegen den zylindrischen Raum des Gewinderohrs abgedichtet ist, wobei die Aufnahme in dem Gewinderohr gleitend gelagert ist.

Dadurch kann die Monomerflüssigkeit durch den zylindrischen Raum gleitet werden, ohne dass sie nach außen austreten kann.

Alternativ kann vorgesehen sein, dass das Gewinderohr an seiner Rückseite eine zylindrische Durchführung aufweist, die gegen die Aufnahme abgedichtet ist, wobei die Aufnahme in der Durchführung gleitend gelagert ist.

Auch hierdurch kann die Monomerflüssigkeit durch den zylindrischen Raum gleitet werden, ohne dass sie nach außen austreten kann.

Ferner kann vorgesehen sein, dass der Mischstab durch einen Druck auf den am Kartuschenkopf anliegenden Mischer und/oder durch ein Drehen oder Schrauben des Gewinderohrs mit der Aufnahme gegen den gegen eine Drehung im Innenraum gesicherten Mischer von der Aufnahme lösbar ist.

Dadurch kann der Mischstab durch eine Bewegung der Aufnahme gegen den im Bereich des Kartuschenkopfs fixierten Mischer von der Aufnahme gelöst werden. Es bedarf dann keiner separaten Vorrichtung zum Lösen des Mischstabs von der Aufnahme. Der Knochenzementapplikator wird hierdurch im Aufbau vereinfacht.

Es kann auch vorgesehen sein, dass der Austragskolben einteilig mit dem Gewinderohr ausgeführt ist, wobei das Gewinderohr an seiner Vorderseite, die dem Kartuschenkopf zugewandt ist, mit dem Austragskolben abgeschlossen ist.

Durch den einteiligen Aufbau des Gewinderohrs mit dem Austragskolben, ist der Aufbau kostengünstig und zudem kann der Knochenzementapplikator leichter zusammengebaut werden. Ferner kann keine Monomerflüssigkeit an Verbindungsstellen des Gewinderohrs mit dem Austragskolben austreten.

Bevorzugt kann vorgesehen sein, dass der Austragskolben nicht durch eine Bewegung des Mischstabs durch die Durchführung im Austragskolben innerhalb des Innenraums bewegbar ist.

Hiermit wird sichergestellt, dass der Mischer alle Bereiche des Innenraums der Kartusche erreichen kann und dadurch eine gute Durchmischung des Knochenzements erzielt werden kann.

Ferner kann vorgesehen sein, dass der Austragskolben zumindest einen für das Knochenzementpulver undurchlässigen und für die Monomerflüssigkeit und Gase durchlässigen Kanal hat.

Dadurch kann die Monomerflüssigkeit durch den Austragskolben hindurch zum Knochenzementpulver strömen, ohne dass das Knochenzementpulver vorher in Richtung des Monomerflüssigkeitsbehälters gelangen kann. Dadurch wird sichergestellt, dass der Knochenzement nur in dem Innenraum der Kartusche zwischen dem Austragskolben und dem Kartuschenkopf entsteht.

Des Weiteren kann vorgesehen sein, dass die Aufnahme bis zu einem Anschlag in das Gewinderohr hineinschiebbar ist, wobei der Mischstab bei einer Bewegung der Aufnahme bis zum Anschlag nicht von der Aufnahme lösbar ist und der Mischstab durch Einschrauben oder Aufschrauben des Gewinderohrs in die Kartusche von der Aufnahme lösbar ist.

Hiermit kann der Knochenzementapplikator auf einfache Weise bedient werden. Gleichzeitig wird so erreicht, dass sich der Mischstab nicht schon beim Mischen des Knochenzements von der Aufnahme löst, sondern erst beim Zusammenschrauben der Aufnahme mit dem Gewinderohr erfolgt.

Gemäß einer bevorzugten Weiterentwicklung der vorliegenden Erfindung kann vorgesehen sein, dass ein Verbindungsmittel, insbesondere eine Fixierungskappe, vorgesehen ist, mit dem die Aufnahme an dem Gewinderohr fixierbar ist, wobei vorzugsweise das Verbindungsmittel über ein hinteres Innengewinde mit einem kleineren Durchmesser als das Gewinde des Gewinderohrs mit einem Außengewinde einer Hülse einer Öffnungseinrichtung verbunden ist, wobei die Hülse im Inneren der Aufnahme auf den Monomerflüssigkeitsbehälter drückbar ist, um den Monomerflüssigkeitsbehälter zu öffnen, und wobei die Fixierungskappe ein vorderes Innengewinde aufweist, das zu dem Gewinde des Gewinderohrs passt.

Hiermit lässt sich die Aufnahme, vorzugsweise mit Hilfe der vorhandenen Öffnungseinrichtung, mit dem Gewinderohr verbinden und dabei der Mischstab von der Aufnahme lösen, was den Aufbau des Knochenzementapplikators vereinfacht und dessen Anwendbarkeit erleichtert.

Gemäß einer bevorzugten Weiterbildung der vorliegenden Erfindung kann vorgesehen sein, dass an der Aufnahme eine von außen bedienbare Öffnungseinrichtung angeordnet ist, mit der der Monomerflüssigkeitsbehälter im Inneren der Aufnahme zu öffnen ist.

Hierdurch kann der Monomerflüssigkeitsbehälter von außen aber gleichzeitig innerhalb der Aufnahme geöffnet werden. Dadurch kann verhindert werden, dass der Anwender mit der Monomerflüssigkeit in Kontakt kommt und dass Monomerflüssigkeit verloren geht, die zur Herstellung des Knochenzements vorgesehen ist und benötigt wird.

Ferner kann vorgesehen sein, dass der Monomerflüssigkeitsbehälter durch Einschieben oder Einschrauben der Öffnungseinrichtung in die Aufnahme zu öffnen ist, wobei sich der Monomerflüssigkeitsbehälter im Inneren der Aufnahme befindet.

Dadurch kann der gesamte Knochenzementapplikator durch ineinander schieben und/oder ineinander schrauben seiner Teile, nämlich der Öffnungseinrichtung in die Aufnahme und der Aufnahme mit dem Gewinderohr und das Gewinderohr in die Kartusche, bedient werden. Dadurch ist der Knochenzementapplikator besonders einfach anwendbar.

Dabei kann vorgesehen sein, dass der Monomerflüssigkeitsbehälter eine Ampulle aus Glas oder einem Kunststoff ist, wobei die Ampulle einen Ampullenkopf, einen zylindrischen Ampullenkörper und einen dem Ampullenkopf gegenüberliegenden Ampullenboden aufweist, wobei der Ampullenkopf einen kleineren Durchmesser als der Ampullenkörper hat und über Schultern mit dem Ampullenkörper verbunden ist, wobei die Öffnungseinrichtung eine Hülse aufweist, die beim Einschieben oder Einschrauben auf die Schultern der Ampulle drückt.

Die Hülse ist vorzugsweise durch einen Hohlzylinder realisiert, wobei eine Öffnung für einen Gasaustausch vorgesehen sein kann, um einen Überdruck in dem Knochenzementapplikator zu vermeiden.

Mit der Hülse kann ein gleichmäßiger Druck auf die Ampulle ausgeübt werden und so ein reproduzierbares Öffnen der Ampulle erreicht werden.

Dadurch, dass die Öffnungseinrichtung eine Hülse aufweist, die beim Einschieben oder Einschrauben auf die Schultern der Ampulle drückt, wird der Ampullenboden auf einen Vorsprung im Inneren der Aufnahme gedrückt und die Ampulle dabei am Ampullenboden geöffnet, so dass die Monomerflüssigkeit ausfließt.

Dabei kann vorgesehen sein, dass die Hülse der Öffnungseinrichtung an der dem Kartuschenkopf gegenüberliegenden Seite aus der Aufnahme herausragt, wobei vorzugsweise die Hülse dabei derart weit aus der Aufnahme herausragt, dass ein Aufbrechen der Ampulle durch vollständiges Einschieben oder Einschrauben der Hülse in die Aufnahme sichergestellt ist.

Hierdurch kann die Hülse besonders einfach in die Aufnahme gedrückt und dadurch die Ampulle geöffnet werden.

Des Weiteren kann vorgesehen sein, dass eine Fixierungskappe der Öffnungseinrichtung ein Außengewinde aufweist und die Aufnahme ein dazu passendes rückseitiges Innengewinde und die Fixierungskappe einen Anschlag für die Aufnahme bildet.

Durch diesen Aufbau wird ein besonders kompakter Knochenzementapplikator bereitgestellt, der einfach und zuverlässig bedienbar ist.

Es kann auch vorgesehen sein, dass zumindest eine Begasungsöffnung in der Wandung der Aufnahme vorgesehen ist, die das Innere der Aufnahme mit der Umgebung des Knochenzementapplikators verbindet, wobei die zumindest eine Begasungsöffnung durch das Einschieben oder Einschrauben der Öffnungseinrichtung verschließbar ist, insbesondere durch Einschieben oder Einschrauben der Hülse verschließbar ist.

Durch die Begasungsöffnungen kann das Innere der Aufnahme und durch eine Verbindung auch der Innenraum der Kartusche und das Innere des Gewinderohrs beziehungsweise der zylindrische Raum des Gewinderohrs des Knochenzementapplikators mit einem sterilisierenden Gas wie Ethylenoxid sterilisiert werden. Gleichzeitig wird die Begasungsöffnung vor dem Öffnen des Monomerflüssigkeitsbehälters mit der Hülse verschlossen, so dass keine Monomerflüssigkeit durch die Begasungsöffnungen nach außen dringen kann.

Ferner kann vorgesehen sein, dass das Innere der Aufnahme mit einem Innenraum der Kartusche flüssigkeitsdurchlässig verbunden ist, wobei bevorzugt hierzu die dem Kartuschenkopf zugewandte Vorderseite der Aufnahme wenigstens einen flüssigkeitsdurchlässigen Durchgang und der Austragskolben wenigstens einen flüssigkeitsdurchlässigen Kanal aufweisen.

Hiermit wird sichergestellt, dass die Monomerflüssigkeit ohne weiteres bei geeigneter Aufstellung des Knochenzementapplikators, nämlich mit dem Kartuschenkopf nach unten, aus der Aufnahme in das Gewinderohr und aus dem Gewinderohr in den Innenraum der Kartusche zwischen den Austragskolben und den Kartuschenkopf fließen kann.

Bevorzugt ist das Innere der Aufnahme mit dem Innenraum der Kartusche flüssigkeitsdurchlässig aber für das Knochenzementpulver undurchlässig verbunden, wobei besonders bevorzugt der Austragskolben wenigstens einen flüssigkeitsdurchlässigen und für das Knochenzementpulver undurchlässigen Kanal aufweist. Hierzu ist besonders bevorzugt eine Porenscheibe am oder im Austragskolben angeordnet.

Bevorzugt kann auch vorgesehen sein, dass der Kartuschenkopf ein auf die Kartusche aufschraubbarer Kartuschendeckel ist, wobei der Kartuschendeckel den Innenraum der Kartusche an deren Vorderseite gasdicht und flüssigkeitsdicht abdichtet und wobei die Austragsöffnung in dem Kartuschendeckel angeordnet ist, vorzugsweise in einem Stutzen im Kartuschendeckel angeordnet ist.

Hierdurch kann der Knochenzementapplikator besonders einfach und kostengünstig zusammengebaut werden. Andere Teile des Knochenzementapplikators können so in die ansonsten zylindrische Kartusche eingesetzt werden, bevor der Kartuschenkopf die Kartusche abschließt.

Gemäß eine bevorzugten Weiterbildung der Erfindung kann vorgesehen sein, dass an der ins Innere der Aufnahme weisenden Seite der Aufnahme ein Dorn zum Öffnen des Monomerflüssigkeitsbehälters angeordnet ist.

Hiermit kann der Monomerflüssigkeitsbehälter an einer definierten Stelle innerhalb der Aufnahme geöffnet werden.

Dabei kann vorgesehen sein, dass der Mischstab bis in den Dorn reicht und sich der Mischstab beim Lösen des Mischstabs von der Aufnahme durch den Dorn hindurch drückt oder der Dorn eine Verlängerung des Mischstabs ist und sich beim Lösen des Mischstabs von der Aufnahme auch der Dorn von der Aufnahme trennt.

Durch diese beiden Maßnahmen kann der Mischstab beim Austragen des Knochenzements aus dem Innenraum der Kartusche zuverlässig in die Aufnahme gedrückt werden, ohne dass sich der Mischstab dabei in der Aufnahme verklemmt, wie beispielsweise an Bruchstücken des geöffneten Monomerflüssigkeitsbehälters.

Es kann also vorgesehen sein, dass der Mischstab in die Aufnahme innerhalb eines in das Innere der Aufnahme weisenden Dorns derart angeordnet ist, dass der Mischstab durch den Dorn hindurch in das Innere der Aufnahme drückbar ist.

Hierdurch wird der Mischstab gezielt durch die mit dem Dorn erzeugte Öffnung in dem Monomerflüssigkeitsbehälter in den Monomerflüssigkeitsbehälter eingeschoben. Bevorzugt ist der Mischstab hierzu aus einem härteren Material gefertigt als der Dorn und die Aufnahme. Beispielsweise kann der Mischstab aus Metall bestehen und der Dorn mit der Aufnahme aus einem Kunststoff.

Es kann vorgesehen sein, dass der Mischstab in seiner Verbindung zur Aufnahme eine Kreisscheibe mit einem Außengewinde aufweist, wobei die Kreisscheibe in ein passendes Innengewinde an der dem Kartuschenkopf zugewandten Vorderseite der Aufnahme geschraubt ist, wobei bevorzugt das Außengewinde der Kreisscheibe und das Innengewinde an der Vorderseite der Aufnahme Linksgewinde sind.

Hierdurch lässt sich der Mischstab mit der Kreisscheibe von der Vorderseite der Aufnahme durch eine Linksdrehung trennen und der Mischstab mit der Kreisscheibe in das Innere der Aufnahme drücken, wenn das Gewinderohr mit der Aufnahme in die Kartusche geschraubt wird.

Die der vorliegenden Erfindung zugrundeliegenden Aufgaben werden auch gelöst durch ein Verfahren zur Herstellung eines Knochenzements mit einem erfindungsgemäßen Knochenzementapplikator umfassend die folgenden Schritte:
A) Öffnen des Monomerflüssigkeitsbehälters im Inneren der Aufnahme und Ausfließen der Monomerflüssigkeit aus dem Monomerflüssigkeitsbehälter, wobei die Monomerflüssigkeit aus der Aufnahme in das Knochenzementpulver im Innenraum der Kartusche fließt,
B) abwechselndes Herausziehen der Aufnahme aus dem Gewinderohr und Einschieben der Aufnahme in das Gewinderohr, wobei der an der Vorderseite der Aufnahme lösbar befestigte Mischstab durch die Durchführung in dem Austragskolben bewegt wird, wobei durch diese Bewegung der Mischer in dem Innenraum der Kartusche bewegt wird und dadurch das Knochenzementpulver und die Monomerflüssigkeit miteinander zum Knochenzement gemischt werden,
C) Lösen des Mischstabs von der Aufnahme durch Aufschrauben oder Einschrauben des Gewinderohrs mit der Aufnahme in den Innenraum der Kartusche,
D) Öffnen der Austragsöffnung, und
E) Auspressen des Knochenzements aus dem Innenraum der Kartusche durch die geöffnete Austragsöffnung, wobei der Knochenzement mit dem Austragskolben aus dem Innenraum der Kartusche gepresst wird und der Austragskolben durch Aufschrauben des Gewinderohrs auf die Kartusche oder durch Einschrauben des Gewinderohrs in die Kartusche angetrieben wird und wobei beim Auf- oder Einschrauben des Gewinderohrs der Mischstab in die Aufnahme hineingedrückt wird.

Bei dünnflüssigeren Knochenzementen kann die Aufnahme zuerst aus dem Gewinderohr herausgezogen werden und dadurch der Mischer zunächst von dem Kartuschenkopf weg in Richtung der Rückseite des Innenraums der Kartusche gezogen werden. Bei hochviskosen Knochenzementen muss zunächst die Aufnahme in das Gewinderohr eingeschoben werden und dabei der Mischer zunächst von der Rückseite aus in Richtung des Kartuschenkopfs gedrückt werden. Hierdurch wird verhindert, dass sich beim Einleiten der Monomerflüssigkeit im Bereich der Einmündung eine stabile Gelschicht als Reaktionsprodukt des Knochenzementpulvers und der Monomerflüssigkeit bildet, die von nachfließender Monomerflüssigkeit nicht mehr durchdrungen werden kann.

Es kann bevorzugt vorgesehen sein, dass der Austragskolben mit einer dem Kartuschenkopf zugewandten Vorderseite des Gewinderohrs fest verbunden ist.

Dabei kann vorgesehen sein, dass der Austragskolben wenigstens einen für das Knochenzementpulver undurchlässigen und für die Monomerflüssigkeit und Gase durchlässigen Kanal aufweist, wobei in Schritt A) die Monomerflüssigkeit durch den Austragskolben hindurch in den Innenraum der Kartusche fließt und in Schritt E) der Austragskolben durch Einschrauben des Gewinderohrs in die Kartusche oder Aufschrauben des Gewinderohrs auf die Kartusche in Richtung des Kartuschenkopfs gedrückt wird.

Hierdurch wird der Aufbau des Knochenzementapplikators kostengünstig und das Herstellungsverfahren vereinfacht.

Dabei kann vorgesehen sein, dass ein in dem Knochenzement enthaltenes Gas in Schritt E) beim Eindrücken oder Aufschrauben oder Einschrauben des Gewinderohrs in den Innenraum der Kartusche durch den wenigstens einen Kanal im Austragskolben aus dem Knochenzement herausgepresst wird.

Hierdurch wird der Knochenzement beim Auspressen durch den Austragskolben entgast.

Ferner kann vorgesehen sein, dass der Monomerflüssigkeitsbehälter in Schritt A) durch das Einschieben oder Einschrauben einer Öffnungseinrichtung in die Aufnahme geöffnet wird.

Hiermit wird das Verfahren für den Anwender besonders einfach umsetzbar. Zudem kann so eine definierte Kraft zum Öffnen des Monomerflüssigkeitsbehälters bereitgestellt werden und damit ein reproduzierbares Öffnen des Monomerflüssigkeitsbehälters erreicht werden.

Dabei kann vorgesehen sein, dass der Monomerflüssigkeitsbehälter in Schritt A) auf einen Dorn im Inneren der Aufnahme gedrückt wird und dadurch in der Aufnahme geöffnet wird, wobei vorzugsweise der Monomerflüssigkeitsbehälter eine Ampulle aus Glas oder einem Kunststoff ist und die Ampulle mit dem Dorn an einem Ampullenboden der Ampulle geöffnet wird.

Auch dies dient dazu, den Monomerflüssigkeitsbehälter an einer definierten Stelle zu öffnen und dadurch den Öffnungsvorgang des Monomerflüssigkeitsbehälters reproduzierbar zu machen.

Dabei kann vorgesehen sein, dass in Schritt E) der Dorn mit dem Mischstab in die Aufnahme gedrückt wird oder der Mischstab den Dorn durchstößt und durch den Dorn hindurch in die Aufnahme gedrückt wird.

Hiermit wird sichergestellt, dass der Mischstab ohne Widerstand durch den geöffneten Monomerflüssigkeitsbehälter oder durch dessen Bruchstücke in die Aufnahme und in den geöffneten Monomerflüssigkeitsbehälter hinein drückbar ist.

Ferner kann vorgesehen sein, dass die Aufnahme in Schritt B) linear bewegt wird und nach Schritt B) und vor Schritt C) die Aufnahme an dem Gewinderohr befestigt wird, wobei vorzugsweise die Aufnahme über das Gewinde des Gewinderohrs an dem Gewinderohr befestigt wird und in den Schritten C) und E) die an dem Gewinderohr befestigte Aufnahme in die Kartusche eingeschraubt wird.

Hiermit kann verhindert werden, dass der Mischstab bereits beim Mischen von der Aufnahme gelöst wird. Ferner wird so auch verhindert, dass bereits beim Mischen eine große Kraft auf den Verschluss der Austragsöffnung ausgeübt wird und auch dass bereits vor dem Abschluss des Mischvorgangs Knochenzement aus der Kartusche austritt. Zudem kann so der Knochenzement durch das Schrauben kraftvoll aus dem Innenraum der Kartusche ausgetrieben werden und der Mischstab mit Hilfe der Kraft durch das Einschrauben der Aufnahme in die Kartusche von der Aufnahme gelöst werden.

Es kann auch vorgesehen sein, dass das Innere der Aufnahme vor Schritt A) mit der Umgebung des Knochenzementapplikators gasdurchlässig verbunden ist, wobei das Innere der Aufnahme vor Schritt A) oder während Schritt A) beim Öffnen des Monomerflüssigkeitsbehälters verschlossen wird.

Hiermit kann das Innere der Aufnahme und auch der Innenraum der Kartusche, also der gesamte Knochenzementapplikator einschließlich seines Inhalts mit einem sterilisierenden Gas wie Ethylenoxid sterilisiert werden. Gleichzeitig kann die Monomerflüssigkeit nicht aus der Aufnahme austreten, nachdem der Monomerflüssigkeitsbehälter innerhalb der Aufnahme geöffnet wurde.

Es wird mit der Erfindung auch vorgeschlagen, dass der Knochenzementapplikator vor Schritt A) mit dem Kartuschenkopf nach unten gehalten oder aufgestellt wird, wobei vorzugsweise der Kartuschenkopf zumindest während der Schritte A) und B) nach unten ausgerichtet bleibt, so dass die Monomerflüssigkeit getrieben von der Schwerkraft in den Innenraum der Kartusche fließt.

Hierdurch wird keine zusätzliche Pumpe benötigt, um die Monomerflüssigkeit in den Innenraum der Kartusche zum Knochenzementpulver zu transferieren.

Ferner kann vorgesehen sein, dass beim Einschieben der Aufnahme in das Gewinderohr während Schritt B) ein bis dahin zurückgebliebener Teil der Monomerflüssigkeit in den Innenraum der Kartusche gedrückt wird.

Hierdurch wird erreicht, dass die Monomerflüssigkeit möglichst vollständig in das Knochenzementpulver transferiert wird, um das gewünschte Mischungsverhältnis von Knochenzementpulver zu Monomerflüssigkeit zu erhalten und damit einen Knochenzement mit den gewünschten Eigenschaften zu erzeugen.

Schließlich kann auch vorgesehen sein, dass vor Schritt C) die Aufnahme vollständig in das Gewinderohr eingeschoben wird und der Mischer dadurch im Innenraum der Kartusche am Kartuschenkopf anliegt, wobei in Schritt C) der Mischstab durch Aufschrauben oder Einschrauben des Gewinderohrs mit der Aufnahme in die Kartusche von der Aufnahme gelöst wird und in Schritt E) die Aufnahme hinein gedrückt wird.

Hiermit kann der Mischstab auf einfache Weise kraftvoll von der Aufnahme gelöst werden.

Der Erfindung liegt die überraschende Erkenntnis zugrunde, dass es durch den von der Aufnahme lösbaren Mischstab und den in die Aufnahme versenkbaren Mischstab gelingt, einen Knochenzementapplikator bereitzustellen, bei dem der Mischstab nicht aus dem Knochenzementapplikator herausgezogen werden muss und bei dem der Mischstab nicht abgebrochen und entfernt werden muss, wenn der Knochenzement mit dem Knochenzementapplikator ausgetragen wird. Die Aufnahme, in der der Monomerflüssigkeitsbehälter angeordnet ist, kann dabei überraschend zur Aufnahme des Mischstabs verwendet werden. So behindert der Mischstab nicht die Bewegung des Austragskolbens beim Auspressen des Knochenzements. Zudem wird in dem Inneren des Gewinderohrs ein Raum bereitgestellt, in dem die Aufnahme unabhängig von der Kartusche bewegt werden kann, wobei gleichzeitig der Mischer durch die Bewegung der Aufnahme in dem Innenraum der Kartusche zum Durchmischen des Knochenzements manuell bedient wird.

Der Mischstab wird nach der Öffnung des Monomerflüssigkeitsbehälters in den hohlen, von der Monomerflüssigkeit geleerten Monomerflüssigkeitsbehälter innerhalb der Aufnahme geschoben, da der Mischstab und der Monomerflüssigkeitsbehälter hintereinander in dem Knochenzementapplikator angebracht sind. Der erfindungsgemäße Knochenzementapplikator ist ein Prepack-Mischsystem und kann ohne vorherige Montageschritte bedient werden. Es ist keine äußere Vakuumquelle für den Monomertransfer notwendig. Der Austrag des Knochenzements erfolgt durch eine manuelle Schraubbewegung des Gewinderohrs mit der darin fixierten hohlzylinderförmigen Aufnahme, wobei das Gewinderohr an seiner dem Kartuschenkopf zugewandten Vorderseite den Austragskolben bildet oder das den Austragskolben im Innenraum der Kartusche antreibt. Die Schraubbewegung entwickelt genügend Auspresskraft, um auch einen hochviskosen Knochenzement aus der Kartusche auspressen zu können und auch um den Mischstab von der Aufnahme zu lösen. Die Bauteile des Knochenzementapplikators können im Wesentlichen durch Kunststoffspritzgießen hergestellt werden und bestehen vorzugsweise aus kostengünstigem thermoplastischem Kunststoff. Die O-Ringe bestehen aus in der Medizintechnik üblichen Elastomeren, wie Silikon oder EPDM (Terpolymere aus Ethylen, Propylen und einem Dien).

Ein beispielhafter erfindungsgemäßer Knochenzementapplikator, der zum Lagern, Mischen und Applizieren vorgesehen ist, ist zusammengesetzt aus
a) einer hohlzylinderförmigen Kartusche, wobei an einem vorderen Ende der Kartusche ein Befestigungsmittel für einen Kartuschendeckel (als Kartuschenkopf) angeordnet ist und wobei am entgegengesetzten rückseitigen Ende der Kartusche an der Kartuscheninnenwand ein Innengewinde angeordnet ist,
b) einem Kartuschendeckel, der mit dem Befestigungsmittel am vorderen Ende der Kartusche gasdicht und flüssigkeitsdicht verbunden ist, wobei der Kartuschendeckel eine Austragsöffnung besitzt,
c) einem Verschlussstopfen, der in der Austragsöffnung des Kartuschendeckels gasdicht und lösbar angeordnet ist,
d) einem bereichsweise hohlzylinderförmigen Ampullenhalter als Aufnahme, der zumindest in einem hinteren Abschnitt ein Innengewinde aufweist,
e) einem Verschluss an der Vorderseite des Ampullenhalters, der den hohlzylinderförmigen Ampullenhalter an einer Längsseite verschließt, wobei ein Mischstab mit einem Mischer an der dem Kartuschenkopf zugewandten Seite des Verschlusses lösbar angebracht ist und wobei die gegenüberliegende Seite des Verschlusses mit einem Stechdorn verbunden ist,
f) einem Gewinderohr, dessen Vorderseite einen axial in der Kartusche verschiebbaren Austragskolben bildet, wobei das Gewinderohr ein Außengewinde hat, das in das Innengewinde am rückseitigen Ende der Kartusche eingeschraubt ist, wobei der Austragskolben für Gase und Flüssigkeiten permeabel und für Knochenzementpulverpartikel impermeabel ist und der zwischen dem Mischer und dem Verschluss des Ampullenhalters in der Kartusche angeordnet ist,
g) einem Monomerflüssigkeitsbehälter mit Monomerflüssigkeit, der mit seiner Bodenseite über dem Stechdorn beabstandet in dem Ampullenhalter angeordnet ist,
h) einer verschiebbaren Hülse als Teil einer Öffnungseinrichtung, die oberhalb des Monomerflüssigkeitsbehälters in dem hohlzylinderförmigen Ampullenhalter derart axial verschiebbar angeordnet ist, dass die Hülse über den Rand des hohlzylinderförmigen Ampullenhalters herausragt, wobei die Hülse ein Außengewinde hat, mit dem die Hülse in das Innengewinde des Ampullenhalters geschraubt ist,
i) eine Fixierungskappe, die mit einem ersten hinteren Innengewinde auf das Außengewinde des hohlzylinderförmigen Ampullenhalters geschraubt ist und die mit einem zweiten vorderen Innengewinde mit einem größeren Durchmesser als das erste Innengewinde auf das Außengewinde des Gewinderohrs schraubbar ist,
j) optional mindestens einer Belüftungsöffnung in der Mantelfläche des hohlzylinderförmigen Ampullenhalters, wobei die Belüftungsöffnung durch axiale Verschiebung der Hülse gasdicht verschließbar ist,
k) einem Knochenzementpulver, das in einem Innenraum der Kartusche angeordnet ist, der durch die Innenwand der Kartusche, den Kartuschendeckel und den Austragskolben gebildet wird,
l) wobei der hohlzylinderförmige Ampullenhalter in das dem Austragskolben gegenüberliegende offene Ende des Gewinderohrs gesteckt ist, so dass der Ampullenhalter in dem Gewinderohr axial beweglich ist, und
m) zumindest der Mischstab nach Öffnung des Monomerflüssigkeitsbehälters in den Hohlraum des Ampullenhalters oder des Monomerflüssigkeitsbehälters verschiebbar ist.

Vorteilhaft ist es, wenn der Verschluss mit dem Mischstab und dem Stechdorn einteilig ausgebildet ist. Dadurch kann der Montageaufwand im Vergleich zu einem zweiteiligen oder dreiteiligen Verschluss mit Mischstab und Stechdorn deutlich gesenkt werden. Der einteilige Verschluss mit Mischstab und Stechdorn kann vorteilhaft durch Kunststoffspritzgießen gefertigt werden.

Es kann auch vorgesehen sein, dass der Verschluss in dem hohlzylinderförmigen Ampullenhalter durch Presspassung lösbar fixiert ist. Der Verschluss kann dabei konisch sein und in einem konischen Sitz des hohlzylinderförmigen Ampullenhalters gelagert sein. Der Konus des Verschlusses verjüngt sich in Richtung des Kartuschenkopfs. Bei Bewegung des hohlzylinderförmigen Ampullenhalters in Richtung des Kartuschenkopfs stützt sich der Mischstab mit Mischelementen auf der Innenseite des Kartuschendeckels ab und drückt den konischen Verschluss aus seinem Sitz. Der Stechdorn mit dem Verschluss und dem Mischstab treten dann in das Innere des Ampullenhalters und in den geöffneten Monomerflüssigkeitsbehälter ein.

In einer anderen beispielhaften Ausgestaltungsvariante besitzt ein innerer Teil des Verschlusses in dem hohlzylinderförmigen Ampullenhalter ein Außengewinde, das in ein Innengewinde des hohlzylinderförmigen Ampullenhalters eingeschraubt ist, wobei der innere Teil des Verschlusses bevorzugt ein linksgängiges Außengewinde besitzt. Beim Drehen des hohlzylinderförmigen Ampullenhalters bewegt sich dieser in Richtung Kartuschenkopf. Der Mischstab mit den Mischelementen wird an die Innenseite des Deckels gepresst. Mit steigendem Anpressdruck an den Deckel kann der Mischstab sich nicht mehr mit dem Ampullenhalter mitdrehen. Der innere Teil des Verschlusses wird dadurch aus dem Innengewinde des hohlzylinderförmigen Ampullenhalters gedreht. Der innere Teil des Verschlusses verlässt seinen Sitz in dem hohlzylinderförmigen Ampullenhalter und wird zusammen mit dem Stechdorn und dem Mischstab in den geöffneten Monomerflüssigkeitsbehälter geschoben.

In einer anderen Ausgestaltungsvariante ist der Mischstab in den Verschluss eingepresst und durchdringt nach Öffnung des Monomerflüssigkeitsbehälters den Verschluss und den Stechdorn. Danach wird der Mischstab in den Ampullenhalter eingeschoben.

Es kann ferner vorgesehen sein, dass der hohlzylinderförmige Ampullenhalter an seiner zylinderförmigen Kopfseite einen Durchmesser gleich oder kleiner dem Innendurchmesser der hohlzylinderförmigen Kartusche hat und der hohlzylinderförmige Ampullenhalter mit seiner Kopfseite gasdicht axial in der Kartusche bewegt werden kann.

Es kann ferner vorgesehen sein, dass die Hülse als Hohlzylinder ausgebildet ist, wobei der Zylindermantel der Hülse auf dem Monomerflüssigkeitsbehälter aufliegt.

Des Weiteren kann vorgesehen sein, dass der innere Teil des Verschlusses einen kleineren Außendurchmesser besitzt als der Innendurchmesser des Monomerflüssigkeitsbehälters. Dadurch kann der innere Teil des Verschlusses mit dem Stechdorn und dem Mischstab problemlos in das Innere des geöffneten Monomerflüssigkeitsbehälters geschoben werden.

Ein beispielhaftes erfindungsgemäßes Verfahren zur Vermischung und Applizieren von Polymethylmethacrylat-Knochenzement mit einem erfindungsgemäßen Knochenzementapplikator kann beispielsweise mit den folgenden nacheinander ablaufenden Schritten realisiert werden:
a) senkrechte Positionierung des Knochenzementapplikators mit dem Kartuschenkopf nach unten,
b) Schrauben der Öffnungseinrichtung in in Richtung Kartuschenkopf,
c) Verschieben der Hülse in Richtung Kartuschenkopf,
d) Optional: Verschluss der mindestens einen Begasungsöffnung in dem hohlzylinderförmigen Ampullenhalter durch die Hülse,
e) Verschieben des Monomerflüssigkeitsbehälters in Richtung Stechdorn durch die axiale Verschiebung der Hülse,
f) Zerstörung des Bodens des Monomerflüssigkeitsbehälters durch den Stechdorn,
g) Ausfließen der Monomerflüssigkeit durch den Verschluss und den für Gase und Flüssigkeiten permeablen Austragskolben in den Innenraum der Kartusche zum Knochenzementpulver,
h) Zurückziehen des hohlzylinderförmigen Ampullenhalters im Gewinderohr entgegengesetzt zum Kartuschenkopf bei gleichzeitiger Rückwärtsbewegung des Mischstabs unter Mischung des Knochenzementpulvers mit der Monomerflüssigkeit,
i) Vorwärtsbewegung des Ampullenhalters im Gewinderohr, Transfer der restlichen Monomerflüssigkeit durch den Überdruck oberhalb der Monomerflüssigkeit durch den Verschluss und den für Gase und Flüssigkeiten permeablen Austragskolben, bei gleichzeitiger Rückwärtsbewegung des Mischstabs unter Mischung des Knochenzementpulvers mit der Monomerflüssigkeit,
j) Mehrmaliges Wiederholen der Schritte h) und i) zur Bildung des Knochenzements aus der Mischung des Polymethylmethacrylat-Knochenzementpulvers mit der Monomerflüssigkeit,
k) Befestigen der Aufnahme an dem Gewinderohr durch Aufschrauben der Fixierungskappe auf das Gewinderohr,
l) Entfernen des Verschlussstopfens aus der Austragsöffnung,
m) Schrauben des Gewinderohrs in der Kartusche in Richtung des Kartuschenkopfs, wobei der Mischstab mit den Mischelementen auf der Innenseite des Deckels (des Kartuschenkopfs) aufsetzt und den inneren Teil des Verschlusses aus seinem konischen Sitz in dem hohlzylinderförmigen Ampullenhalter in Richtung Kartuschenboden herausdrückt,
n) Einschieben des Verschlusses mit Stechdorn und Mischstab in den geöffneten Monomerflüssigkeitsbehälter, und
o) Herauspressen des Polymethylmethacrylat-Knochenzements durch die Schraubbewegung des Gewinderohrs in Richtung Kartuschenkopf.

Ein beispielhaftes alternatives Verfahren zur Vermischung und Applizieren von Polymethylmethacrylat-Knochenzement mit dem erfindungsgemäßen Knochenzementapplikator kann durch folgende nacheinander ablaufende Schritte charakterisiert sein:
a) senkrechte Positionierung des Knochenzementapplikators mit dem Kartuschenkopf nach unten,
b) Schrauben der auf den hohlzylinderförmigen Ampullenhalter als Aufnahme angeschraubten Fixierungskappe in Richtung Kartuschenkopf,
c) Verschieben der Hülse in Richtung Kartuschenkopf durch die Fixierungskappe,
d) Verschluss der mindestens einen Begasungsöffnung in dem hohlzylinderförmigen Ampullenhalter durch die Hülse,
e) Verschieben des Monomerflüssigkeitsbehälters in Richtung Stechdorn durch die axiale Verschiebung der Hülse,
f) Zerstörung des Bodens des Monomerflüssigkeitsbehälters durch den Stechdorn,
g) Ausfließen der Monomerflüssigkeit durch den Verschluss und den für Gase und Flüssigkeiten permeablen Austragskolben in den vorderen Teil des Innenraums der Kartusche zum Knochenzementpulver,
h) Zurückziehen des hohlzylinderförmigen Ampullenhalters im Gewinderohr entgegengesetzt zum Kartuschenkopf bei gleichzeitiger Rückwärtsbewegung des Mischstabs unter Mischung des Knochenzementpulvers mit der Monomerflüssigkeit,
i) Vorwärtsbewegung des Ampullenhalters im Gewinderohr, Transfer der restlichen Monomerflüssigkeit durch den Überdruck oberhalb der Monomerflüssigkeit durch den Verschluss und den für Gase und Flüssigkeiten permeablen Austragskolben, bei gleichzeitiger Rückwärtsbewegung des Mischstabs unter Mischung des Knochenzementpulvers mit der Monomerflüssigkeit,
j) Mehrmaliges Wiederholen der Schritte h) und i) zur Bildung des Knochenzements aus der Mischung des Polymethylmethacrylat-Knochenzementpulvers mit der Monomerflüssigkeit,
k) Befestigen der Aufnahme an dem Gewinderohr durch Aufschrauben der Fixierungskappe auf das Gewinderohr,
l) Entfernen des Verschlussstopfens aus der Austragsöffnung,
m) Schrauben des Gewinderohrs in der Kartusche in Richtung des Kartuschenkopfs, wobei der Mischstab mit den Mischelementen auf der Innenseite des Deckels aufsetzt und den inneren Teil des Verschlusses mit seinem Außengewinde aus dem Innengewinde des hohlzylinderförmigen Ampullenhalters in Richtung Kartuschenboden herausschraubt,
n) Einschieben des Verschlusses mit Stechdorn und Mischstab in den geöffneten Monomerflüssigkeitsbehälter, und
o) Herauspressen des Polymethylmethacrylat-Knochenzements durch die Schraubbewegung des Gewinderohrs in Richtung Kartuschenkopf.

Ein weiteres beispielhaftes alternatives Verfahren zur Vermischung und Applizieren von Polymethylmethacrylat-Knochenzement mit dem erfindungsgemäßen Knochenzementapplikator kann durch folgende nacheinander ablaufende Schritte charakterisiert sein:
a) senkrechte Positionierung des Knochenzementapplikators mit dem Kartuschenkopf nach unten,
b) Schrauben der auf die hohlzylinderförmige Aufnahme angeschraubten Fixierungskappe in Richtung Kartuschenkopf,
c) Verschieben der Hülse in Richtung Kartuschenkopf durch die Fixierungskappe,
d) Verschluss der mindestens einen Begasungsöffnung in der hohlzylinderförmigen Aufnahme durch die Hülse,
e) Verschieben des Monomerflüssigkeitsbehälters in Richtung Stechdorn durch die axiale Verschiebung der Hülse,
f) Zerstörung des Bodens des Monomerflüssigkeitsbehälters durch den Stechdorn,
g) Ausfließen der Monomerflüssigkeit durch den Verschluss und den für Gase und Flüssigkeiten permeablen Austragskolben in den vorderen Teil des Innenraums der Kartusche zum Knochenzementpulver,
h) Zurückziehen der hohlzylinderförmigen Aufnahme im Gewinderohr entgegengesetzt zum Kartuschenkopf bei gleichzeitiger Rückwärtsbewegung des Mischstabs unter Mischung des Knochenzementpulvers mit der Monomerflüssigkeit,
i) Vorwärtsbewegung der Aufnahme im Gewinderohr, Transfer der restlichen Monomerflüssigkeit durch den Überdruck oberhalb der Monomerflüssigkeit durch den Verschluss und den für Gase und Flüssigkeiten permeablen Austragskolben, bei gleichzeitiger Rückwärtsbewegung des Mischstabs unter Mischung des Knochenzementpulvers mit der Monomerflüssigkeit,
j) Mehrmaliges Wiederholen der Schritte h) und i) zur Bildung des Knochenzements aus der Mischung des Polymethylmethacrylat-Knochenzementpulvers mit der Monomerflüssigkeit,
k) Befestigen der Aufnahme an dem Gewinderohr durch Aufschrauben der Fixierungskappe auf das Gewinderohr,
l) Entfernen des Verschlussstopfens aus der Austragsöffnung,
m) Schrauben des Gewinderohrs in der Kartusche in Richtung des Kartuschenkopfs, wobei der Mischstab mit den Mischelementen auf der Innenseite des Deckels aufsetzt und den Verschluss und den Stechdorn durchsticht,
n) Einschieben des Mischstabs in den geöffneten Monomerflüssigkeitsbehälter, und
o) Herauspressen des Polymethylmethacrylat-Knochenzements durch die Schraubbewegung des Gewinderohrs in Richtung Kartuschenkopf.

Das Auspressen des Knochenzements erfolgt durch Vortreiben des Austragskolbens mit dem Gewinderohr, wobei der Ampullenhalter an dem Gewinderohr fixiert ist.

Im Folgenden werden weitere Ausführungsbeispiele der Erfindung anhand von acht schematisch dargestellten Figuren erläutert, ohne jedoch dabei die Erfindung zu beschränken. Dabei zeigt:
Figur 1: eine schematische Querschnittansicht eines beispielhaften erfindungsgemäßen Knochenzementapplikators zum Herstellen eines Knochenzementteigs;
Figur 2: eine schematische perspektivische Außenansicht des Knochenzementapplikators nach Figur 1;
Figur 3: eine schematische Seitenansicht des Knochenzementapplikators nach den Figuren 1 und 2;
Figur 4: eine schematische Querschnittansicht des Knochenzementapplikators nach den Figuren 1 bis 3 mit geöffnetem Monomerflüssigkeitsbehälter zur Verdeutlichung des Ablaufs eines erfindungsgemäßen Verfahrens;
Figur 5: eine schematische Querschnittansicht des Knochenzementapplikators nach den Figuren 1 bis 4 mit in die Kartusche eingeschobener Aufnahme zur Verdeutlichung des Ablaufs eines erfindungsgemäßen Verfahrens;
Figur 6: eine schematische Querschnittansicht des Knochenzementapplikators nach Figur 5 mit an einem Gewinderohr befestigter Aufnahme zur Verdeutlichung des Ablaufs eines erfindungsgemäßen Verfahrens;
Figur 7: eine schematische Querschnittansicht des Knochenzementapplikators nach den Figuren 1 bis 6 mit in die Kartusche eingeschraubter Aufnahme nach Austragen des Knochenzements zur Verdeutlichung des Ablaufs eines erfindungsgemäßen Verfahrens; und
Figur 8: eine schematische perspektivische Querschnittansicht des Knochenzementapplikators nach den Figuren 1 bis 7 ohne die Ausgangskomponenten im Lagerungszustand.

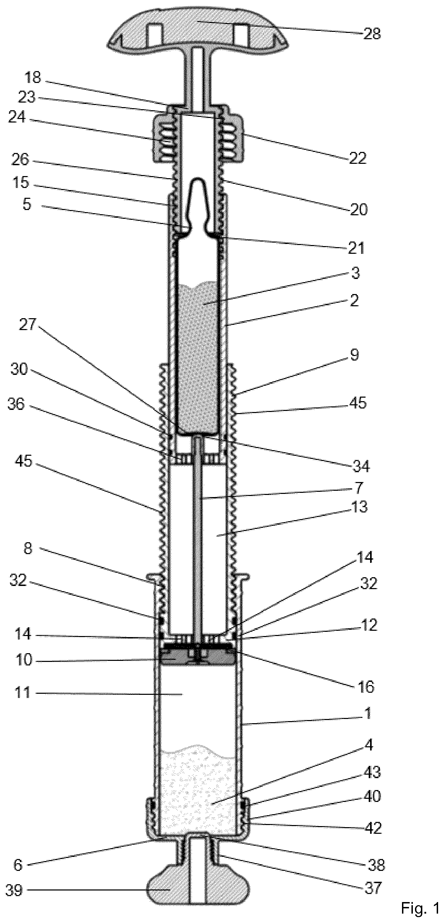

In den Figuren sind Abbildungen eines erfindungsgemäßen Knochenzementapplikators zum Lagern von Ausgangskomponenten 3, 4 eines Knochenzements 48 und zum Mischen des Knochenzements 48 gezeigt. Die Figuren 1 und 4 bis 7 zeigen dabei den Ablauf eines erfindungsgemäßen Verfahrens durchgeführt mit dem erfindungsgemäßen Knochenzementapplikator in Form von fünf Querschnittansichten des Knochenzementapplikators.

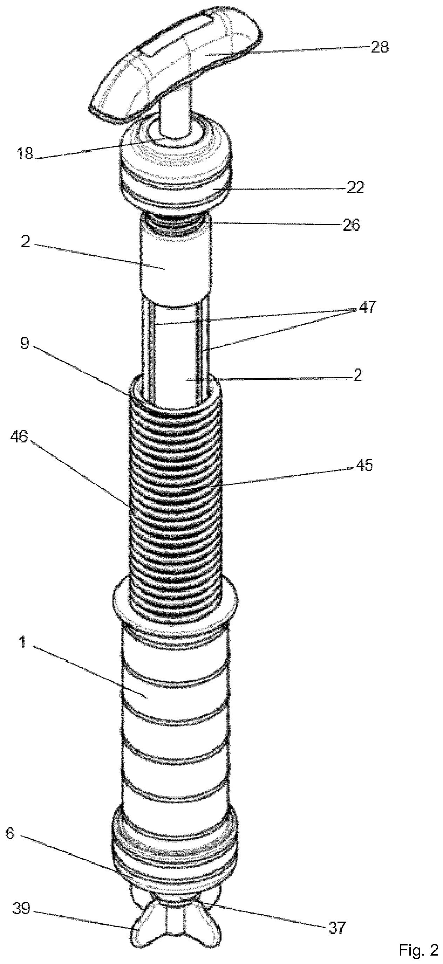

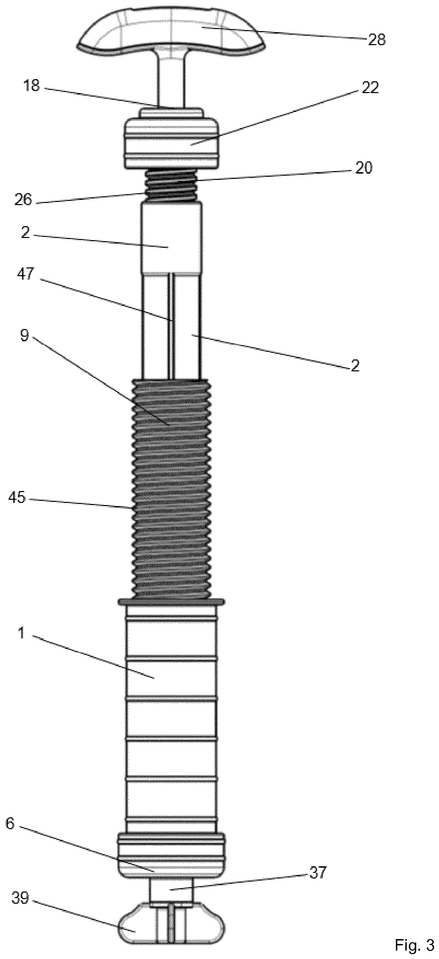

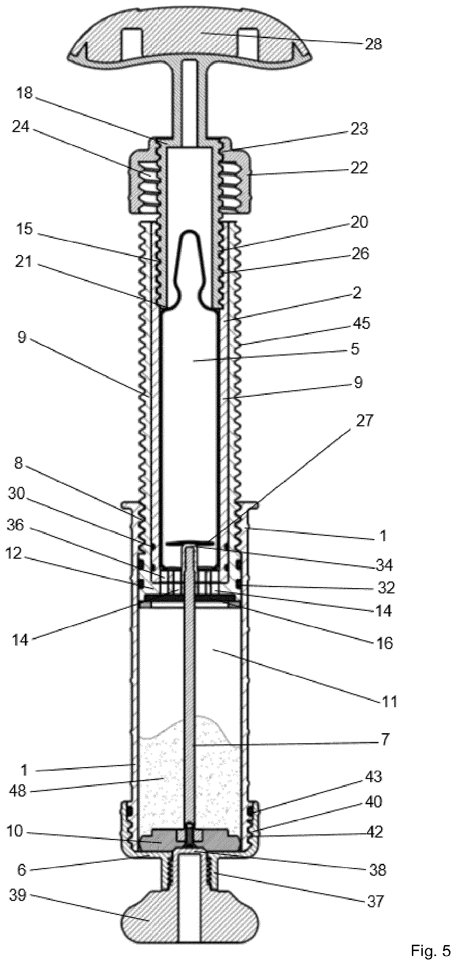

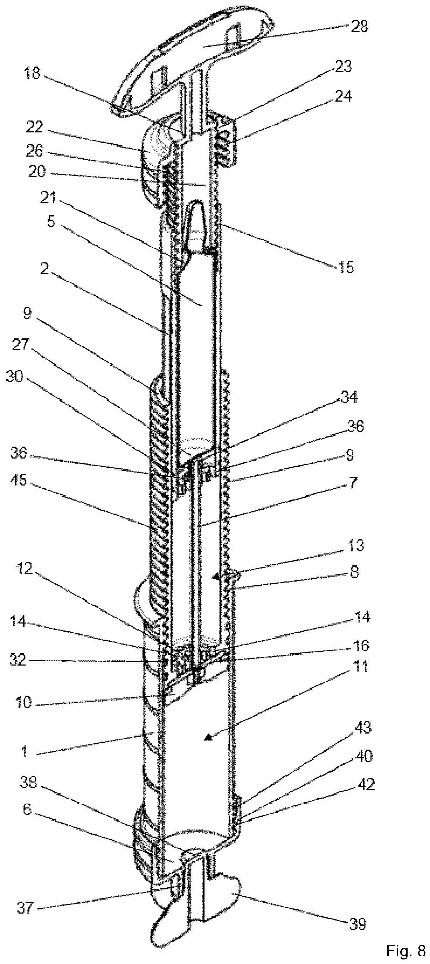

Der erfindungsgemäße Knochenzementapplikator weist eine röhrenförmige Kartusche 1 aus Kunststoff auf, die einen vorderen Teil (in den Figuren 1 bis 8 unten) des Knochenzementapplikators bildet. Ein rückseitiger, hinterer Teil des Knochenzementapplikators wird durch eine Aufnahme 2 gebildet. Der Knochenzementapplikator ist zur Herstellung eines Knochenzements 48 (siehe Figuren 5 bis 7) vorgesehen, der aus einer Monomerflüssigkeit 3 und aus einem Knochenzementpulver 4 hergestellt wird. Die Monomerflüssigkeit 3 und das Knochenzementpulver 4 sind die Ausgangskomponenten 3, 4 des Knochenzements 48. Die Monomerflüssigkeit 3 ist in einer aufbrechbaren Ampulle 5 aus Glas oder aus einem Kunststoff als Monomerflüssigkeitsbehälter für die Monomerflüssigkeit 3 enthalten, wobei die Ampulle 5 in der Aufnahme 2 steckt. Die Kartusche 1 bildet in ihrem Inneren einen zylindrischen Innenraum 11, in dem das Knochenzementpulver 4 enthalten ist. Somit ist der Knochenzementapplikator auch zum Lagern der Monomerflüssigkeit 3 und des Knochenzementpulvers 4 geeignet.

Die Kartusche 1 weist an ihrer Vorderseite (in den Figuren unten) einen Kartuschendeckel 6 als Kartuschenkopf auf. In dem Kartuschendeckel 6 ist eine Austragsöffnung vorgesehen. In der seitlichen Wandung der Aufnahme 2 können sich gemäß einer alternativen Variante des Knochenzementapplikators mehrere Begasungsöffnungen (nicht gezeigt) befinden, durch die ein Gas aus dem Inneren des Knochenzementapplikators abgesaugt werden kann und durch die ein sterilisierendes Gas wie Ethylenoxid eingefüllt werden kann, um das Innere des Knochenzementapplikators zu sterilisieren.

An der Vorderseite der Aufnahme 2 ist ein Mischstab 7 befestigt, der sich von der Vorderseite der Aufnahme 2 bis in den vorderen Teil der Kartusche 1 erstreckt, in dem sich das Knochenzementpulver 4 befindet.

Am rückseitigen Ende der Kartusche 1 befindet sich ein Innengewinde 8. In dieses Inngewinde 8 ist ein Gewinderohr 9 mit einem passenden Außengewinde 45 eingeschraubt. Das Gewinderohr 9 ist an seiner Vorderseite (in den Figuren unten) durch einen Austragskolben 12 verschlossen. Im Inneren des Gewinderohrs 9 ist ein zylindrischer Raum 13 gebildet, der an seiner Vorderseite durch den Austragskolben 12 begrenzt ist und in den die Aufnahme axial beweglich eingesteckt ist.

Die Aufnahme 2 weist an ihrer Rückseite ein Innengewinde 15 auf und weist in ihrem Inneren eine zylindrische Kammer auf, in der die Ampulle 5 steckt. In einem vorderen Bereich ist die Aufnahme 2 außen zylindrisch geformt, wobei vier vorspringende Leisten 47 parallel zur Zylinderachse der Aufnahme 2 an der äußeren Oberfläche der Aufnahme 2 vorgesehen sind. Die Ampulle 5 hat einen zylindrischen Ampullenkörper mit einem zu dem Inneren der Aufnahme 2 passendem Durchmesser. Im Inneren der Kartusche 1 bildet die Kartusche 1 den zylindrischen Innenraum 11. Die Zylindergeometrie des Innenraums 11 und der Kammer der Aufnahme 2 entsprechen Zylindern mit kreisförmiger Grundfläche.

An der Vorderseite des Mischstabs 7 ist ein Mischer 10 in Form von Mischflügeln mit einem umgebenden Abkratzring befestigt. Mit dem Abkratzring können die Bereiche direkt an der Innenwand des Innenraums 11 erreicht werden.

Die Aufnahme 2 ist an ihrer Vorderseite durch eine Wandung mit mehreren Durchgängen 36 als Verschluss der Vorderseite begrenzt, wobei die Wandung an der Vorderseite der Aufnahme 2 die Kammer nach vorne an ihrer kreisförmigen Grundfläche verschließt. Der Austragskolben 12 ist axial beweglich im zylindrischen Innenraum 11 der Kartusche 1 angeordnet. Der Mischstab 7 ist durch einen zentralen Durchgang im Austragskolben 12 geführt, so dass der Mischstab 7 gegen den Austragskolben 12 bewegt werden kann, ohne dass sich dabei der Austragskolben 12 im Innenraum der Kartusche 1 bewegen müsste. Bei zurückgezogener Aufnahme 2 liegt der Mischer 10 an der Vorderseite des Austragskolbens 12 an. Dadurch kann der gesamte Teil des Innenraums 11, der seitlich durch die Kartusche 1, vorne durch den Kartuschendeckel 6 und hinten durch den Austragskolben 12 begrenzt ist, von dem Mischer 10 erreicht werden. Dadurch ist eine vollständige Durchmischung des Knochenzementpulvers 4 mit der Monomerflüssigkeit 3 in diesem Bereich gewährleistet.

Der Austragskolben 12 weist mehrere Kanäle 14 durch den Austragskolben 12 auf, die ringförmig um den zentralen Durchgang für den Mischstab 7 herum in dem Austragskolben 12 angeordnet sind und die Vorderseite des Austragskolbens 12 mit der Rückseite des Austragskolbens 12 und dadurch den Innenraum 11 der Kartusche 1 mit dem Raum 13 des Gewinderohrs 9 verbinden. Die Kanäle 14 sind mit einem ringförmigen Porenfilter 16 abgedeckt. Der Porenfilter 16 ist für das Knochenzementpulver 4 aus dem Innenraum 11 der Kartusche 1 undurchlässig und für die Monomerflüssigkeit 3 und Gase durchlässig. Dadurch wird ein Vordringen des Knochenzementpulvers 4 in den Raum 13 des Gewinderohrs 9 und das Innere der Aufnahme 2 verhindert.

Der Austragskolben 12 weist einen größeren Außendurchmesser auf, als der Innendurchmesser des Innengewindes 8 der Kartusche 1. Der Außendurchmesser des zylindrischen Austragskolbens 12 passt zum Innendurchmesser des Innenraums 11 der Kartusche 1. Der Austragskolben 12 dichtet den Innenraum 11 der Kartusche 1 ab.

An der Rückseite der Aufnahme 2 ist eine Öffnungseinrichtung 18 vorgesehen, mit der die Ampulle 5 in Richtung des Austragskolbens 12 gedrückt werden kann, um die Ampulle 5 im Inneren der Aufnahme 2 zu öffnen, so dass die Monomerflüssigkeit 3 in der Aufnahme 2 ausfließt. Die Öffnungseinrichtung 18 weist hierzu eine Hülse 20 auf, wobei die Hülse 20 einen Hohlzylinder bildet, in dem ein Ampullenkopf der Ampulle 5 angeordnet ist. Die Hülse 20 der Öffnungseinrichtung 18 kann dadurch auf Schultern 21 der Ampulle 5 drücken, um diese nach vorne in Richtung des Austragskolbens 12 zu drücken und dadurch zu öffnen. Da die Hülse 20 auf die Schultern 21 drückt, wird die Kraft durch den Ampullenkörper bis zu einem Ampullenboden 27 der Ampulle 5 geleitet. Die Wandungen des Ampullenkörpers sind sehr stabil, so dass die Ampulle 5 in diesem Bereich nicht brechen wird. Die Ampulle 5 kann so am Ampullenboden 27 aufgebrochen werden.

Die Hülse 20 hat hierzu ein Außengewinde 26, das zu dem Innengewinde 15 der Aufnahme 2 passt. Die Hülse 20 ist in das Innengewinde 15 der Aufnahme 2 geschraubt und kann tiefer in die Aufnahme 2 geschraubt werden, um die Ampulle 5 zu öffnen. Die Hülse 20 deckt die Aufnahme 2 im Bereich der Rückseite des Inneren der Aufnahme 2 ab.

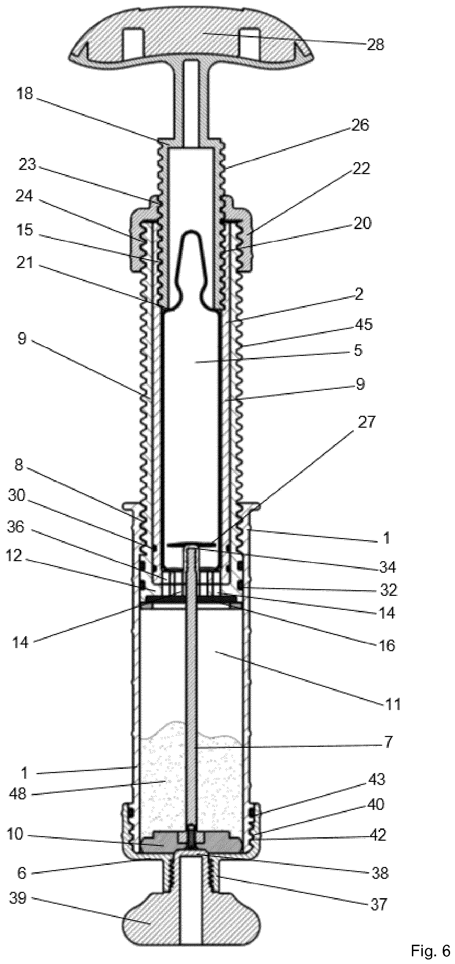

Eine Fixierungskappe 22 ist auf das Außengewinde 26 der Hülse 20 geschraubt. Hierzu hat die Hülse 20 ein passendes hinteres Innengewinde 23. Die Fixierungskappe 22 dient dem Fixieren der Aufnahme 2 an dem Gewinderohr 9. Hierzu hat die Fixierungskappe 22 ein vorderes Innengewinde 24, das auf das Außengewinde 45 des Gewinderohrs 9 passt. Durch Aufschrauben der Fixierungskappe 22 auf das Gewinderohr 9 bei vollständig eingeschobener Aufnahme 2 kann so die Aufnahme 2 in dem Gewinderohr 9 fixiert werden (siehe Figur 6). Hierzu haben die Innengewinde 23, 24 der Fixierungskappe 22, das Außengewinde 45 des Gewinderohrs 9 und das Außengewinde 26 der Hülse 20 alle die gleiche Steigung.

Der Knochenzementapplikator muss für die Anwendung mit dem Kartuschendeckel 6 nach unten gehalten oder aufgestellt werden, so wie das in den Figuren 1 bis 8 gezeigt ist.

Die Öffnungseinrichtung 18 ist ein kleines Stück aber nicht bis zu einem Anschlag in die Rückseite der Aufnahme 2 eingeschraubt und so an dieser befestigt. Es ist wichtig, dass die Öffnungseinrichtung 18 noch weiter in die Aufnahme 2 aufgeschraubt werden kann und dadurch die Hülse 20 tiefer in die Aufnahme 2 eingeschoben werden kann, damit die Ampulle 5 in der Aufnahme 2 geöffnet werden kann.

Um eine Drehung der Fixierungskappe 22 oder der Öffnungseinrichtung 18 in die falsche Richtung zu verhindern, kann eine Rückdrehsicherung vorgesehen sein (in den Figuren 1 bis 8 nicht zu sehen). Die Rückdrehsicherung verhindert ein Lösen der Fixierungskappe 22 beziehungsweise ein Lösen der Öffnungseinrichtung 18 von der Aufnahme 2. Die Rückdrehsicherung kann beispielsweise als Schraubensicherung in Form einer Sperrkantscheibe oder durch ein Keilsicherungsscheibenpaar oder ähnliche Maßnahmen realisiert werden.

Um die Öffnungseinrichtung 18 und die Aufnahme 2 bequem mit der Hand drehen zu können und um die Aufnahme 2 bequem in den Raum 13 des Gewinderohrs 9 einschieben und herausziehen zu können, ist deren rückseitiges Ende mit einem Griff 28 ausgestattet. Um die Aufnahme gegen die Innenwand des Gewinderohrs 9 abzudichten, sind zwei umlaufende Dichtungen 30 aus Gummi in umlaufenden Nuten am vordersten Außenumfang der der Aufnahme angeordnet. Das Innengewinde 15 der Aufnahme ist begrenzt und bildet so einen Anschlag der ein weiteres Aufschrauben der Öffnungseinrichtung 18 in die Aufnahme 2 verhindert.

In gleicher Weise befinden sich am Außenumfang des Austragskolbens 12 zwei in Längsrichtung voneinander beabstandete Nuten, in denen sich zwei umlaufende Dichtungen 32 aus Gummi befinden. Die Dichtungen 32 dichten den Austragskolben 12 gegen den Innenraum 11 der Kartusche 1 ab und trennen den Innenraum 11 der Kartusche 1, in dem das Knochenzementpulver 4 angeordnet ist, von dem Raum des Gewinderohrs 9.

An der zum Kartuschendeckel 6 weisenden vorderen Wandung der Aufnahme 2 ist ein Dorn 34 zum Aufbrechen der Ampulle 5 angeordnet. Der Dorn 34 weist hierzu ins Innere der Aufnahme 2. Die Ampulle 5 kann zum Öffnen der Ampulle 5 mit der Hülse 20 auf den Dorn 34 gedrückt werden, bis der Ampullenboden 27 der Ampulle 5 in den Ampullenkörper gedrückt wird. Der Dorn 34 hat eine stumpfe Spitze, die dazu dient, dass die Kraft auf die Ampulle 5 auf einen mittleren Bereich des Ampullenbodens 27 wirkt, so dass eine Sollbruchstelle in der Verbindung zwischen dem Ampullenboden 27 und den seitlichen Wandungen des Ampullenkörpers genutzt wird. Die Kraft hierzu wird über die Hülse 20 ausgeübt. Die Hülse 20 weist in etwa den gleichen Durchmesser wie der Ampullenkörper der Ampulle 5 auf. Der Ampullenkopf der Ampulle 5 ist dabei im Inneren der Hülse 20 angeordnet. Damit wird erreicht, dass die Ampulle 5 nicht im Bereich der Hülse 20 gebrochen wird, weil der zylindrische Ampullenkörper sehr stabil ist, während der Dorn 34 relativ leicht von vorne in die Ampulle 5 gedrückt werden kann.

Der Mischstab 7 ist innerhalb des Dorns 34 an der Aufnahme 2 befestigt. Der Dorn 34 ist über eine Sollbruchstelle mit der Aufnahme 2 verbunden, so dass bei einem Druck auf den Mischstab 7, der Mischstab 7 den Dorn 34 von der Aufnahme 2 abtrennt und so der Mischstab 7 mit dem Dorn 34 an der Spitze durch die vordere Grundfläche der Aufnahme 2 beweglich ist. Alternativ kann auch eine innere Kreisscheibe (nicht gezeigt) der Vorderseite der Aufnahme 2 über ein Gewinde mit der Aufnahme 2 verbunden sein, so dass der Dorn 34 mit dieser Kreisscheibe durch eine Drehung der Aufnahme 2 gegen den hierzu gegen den Kartuschendeckel 6 fixierten Mischstab 7 von der restlichen Aufnahme 2 getrennt werden kann, so dass der Mischstab 7 wieder gegen die restliche Aufnahme 2 beweglich ist.

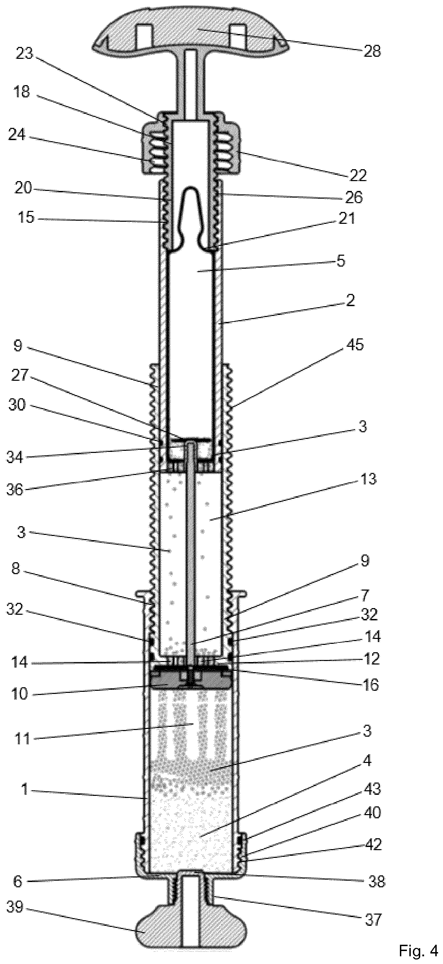

Um den Dorn 34 herum sind mehrere Durchgänge 36 angeordnet, die das Innere der Aufnahme 1 mit dem Innenraum 11 der Kartusche 1 verbinden. Durch die Durchgänge 36 kann die Monomerflüssigkeit 3 in den Innenraum 11 der Kartusche 1 fließen, so wie das in Figur 4 dargestellt ist.

Die Vorderseite der Kartusche 1 ist mit dem Kartuschendeckel 6 verschlossen. In der Mitte des Kartuschendeckels 6 ist ein Stutzen 37 ausgeformt, der die Austragsöffnung im Kartuschendeckel 6 begrenzt. In dem Stutzen 37 ist ein Verschluss 38 eingeschraubt und dadurch lösbar befestigt, der die Austragsöffnung verschließt. Der Verschluss 38 kann über Flügel 39 nach Art einer Flügelschraube bedient werden. Der Kartuschendeckel 6 ist mit einem Innengewinde 40 auf ein Außengewinde 42 an der Vorderseite der Kartusche 1 geschraubt. Der Kartuschendeckel 6 ist zusätzlich über eine umlaufende Dichtung 43 gegen die Kartusche 1 abgedichtet.

In dem vorderen Teil des Innenraums 11 der Kartusche 1 befindet sich der Mischer 10, mit dem der Inhalt des vorderen Teils des Innenraums 11 durch eine manuelle Bewegung des Mischers 10 durchmischt werden kann. Die manuelle Bewegung des Mischers 10 erfolgt durch Einschieben und Herausziehen der Aufnahme 2 in das Gewinderohr 9. Dabei wird nämlich auch der Mischstab 7, der an der Vorderseite der Aufnahme 2 befestigt ist, linear hin- und herbewegt. Der Mischstab 7 bewegt sich dabei durch die Durchführung im Austragskolben 12 und der an dem Mischstab 7 befestigte Mischer 10 bewegt sich in dem Innenraum 11 der Kartusche 1.

Wenn die Aufnahme 2 bis zum Anschlag in das Gewinderohr 9 eingeschoben wird, trifft der Mischer 10 auf den Kartuschendeckel 6, sofern das Gewinderohr 9 bis zu einem durch das Innengewinde 8 der Kartusche 1 und den Förderkolben 12 gebildeten Anschlag maximal aus der Rückseite des Innenraums 11 der Kartusche 1 herausgeschraubt ist. Dabei ist die Länge des Mischstabs 7 genau so gewählt, dass der Mischer 10 genau dann an der Vorderseite des Innenraums 11 am Kartuschendeckel 6 anliegt. Dadurch kann der Knochenzement 48 auch an der Vorderseite des Innenraums 11 vollständig mit dem Mischer 10 erreicht und gemischt werden.

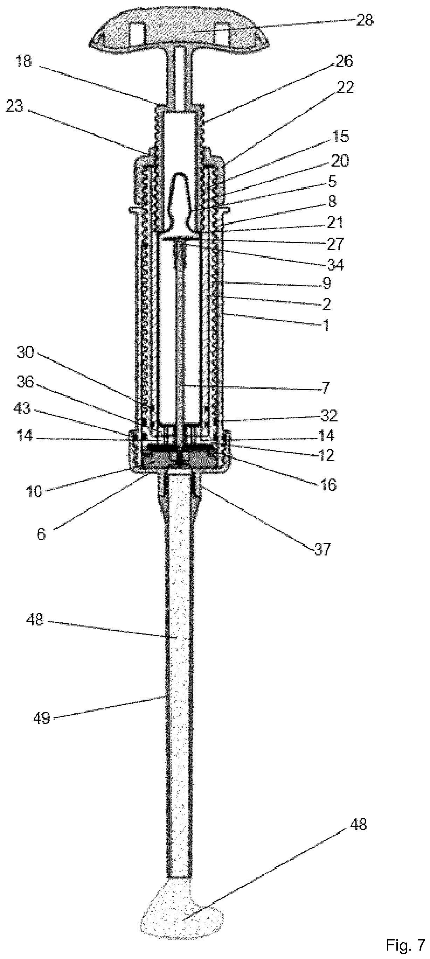

Der Verschluss 38 ragt ein kleines Stück in den Innenraum 11 der Kartusche 1 hinein. An der dem Kartuschendeckel 6 zugewandten Vorderseite des Mischers 10 ist eine Vertiefung vorgesehen, die den in den Innenraum 11 ragenden Teil des Verschlusses 38 aufnimmt. Dadurch kann auch der am Verschluss 38 und am Kartuschendeckel 6 anliegende Knochenzement 48 gemischt werden und gleichzeitig wird durch diese Vertiefung ein freier Strömungsquerschnitt für den Knochenzement 48 bereitgestellt, wenn der Verschluss 38 entfernt ist und der Mischer 10 beim Austragen des Knochenzements 48 am Kartuschendeckel 6 anliegt (siehe Figur 7).

Im Folgenden wird der Ablauf eines erfindungsgemäßen Verfahrens anhand der Figuren 1 bis 8 erläutert. Zunächst befindet sich der Knochenzementapplikator im Ausgangszustand (siehe Figuren 1 bis 3 und 8). In diesem Zustand ist der Knochenzementapplikator verpackt und mit Ethylenoxid sterilisiert worden. Das Ethylenoxid kann durch Zwischenräume in der Öffnungseinrichtung 18 in das Innere der Aufnahme 2 und durch die Durchgänge 36, den Porenfilter 16 und die Kanäle 14 in den Innenraum 11 der Kartusche 1 gelangen. Der Gasaustausch erfolgt dabei in einer Vakuumkammer oder Unterdruckkammer. In diesem Zustand (siehe Figuren 1 bis 3 und 8) wird der Knochenzementapplikator ausgepackt.

Der Knochenzementapplikator wird mit dem Kartuschendeckel 6 nach unten gehalten. Anschließend wird die Öffnungseinrichtung 18 in die Aufnahme 2 geschraubt. Auch hierbei wird der Knochenzementapplikator mit dem Kartuschendeckel 6 nach unten gehalten. Dabei drückt die Hülse 20 die Ampulle 5 an deren Schultern 21 nach unten. Anschließend wird die Ampulle 5 mit dem Ampullenboden 27 auf den Dorn 34 gedrückt und die Ampulle 5 bricht am Ampullenboden 27 auf. Dieser Zustand ist in Figur 4 gezeigt.

Die Monomerflüssigkeit 3 tritt aus der geöffneten Ampulle 5 im Bereich der Durchgänge 36 aus. Da der Knochenzementapplikator mit dem Kartuschendeckel 6 nach unten gehalten wird, fließt die Monomerflüssigkeit 3 getrieben von der Schwerkraft sofort nach unten durch die Durchgänge 36, den Porenfilter 16 und die Kanäle 14 in den Innenraum 11 der Kartusche 1 und verteilt sich in dem Knochenzementpulver 4 (siehe Figur 4). Um den Monomertransfer zu beschleunigen kann die Aufnahme 2 in das Gewinderohr 9 hineingeschoben und herausgezogen werden.

Das Mischen des Knochenzements 48 beziehungsweise der Ausgangskomponenten 3, 4 des Knochenzements 48 erfolgt durch Einschieben und Herausziehen der Aufnahme 2 in dem Raum 13 des Gewinderohrs 9 bei gleichzeitiger Bewegung des Mischers 10 im Innenraum 11 der Kartusche 1. Der Mischer 10 erreicht dabei alle Orte in dem Innenraum 11 zwischen dem Austragskolben 12 und dem Kartuschendeckel 6. Zum Führen dieser Bewegung sind außen an der Aufnahme 2 die Leisten 47 angeordnet. Die Leisten 47 verhindern, dass die Aufnahme 2 beim Mischen wackelt.

Schließlich ist der Knochenzement 48 gemischt und die Aufnahme 2 wird vollständig in das Gewinderohr 9 eingeschoben, so dass der Mischer 10 an dem Kartuschendeckel 6 anliegt. Diese Situation ist in Figur 5 dargestellt.

Damit die Aufnahme 2 mit dem Gewinderohr 9 fixiert ist, so dass der Mischstab 7 in die Aufnahme 2 eingedrückt werden kann, wird die Fixierungskappe 22 auf das Außengewinde 45 des Gewinderohrs 9 aufgeschraubt. Diese Situation ist in Figur 6 dargestellt.

Auf diese Weise kann die Aufnahme 2 zusammen mit dem Gewinderohr 9 in die Kartusche 1 hinein geschraubt werden. Dadurch lässt sich die Aufnahme 2 kraftvoll gegen die Kartusche 1 bewegen. Der Mischer 10 liegt vorne am Kartuschendeckel 6 an, so dass der Mischstab 7 nicht ausweichen kann. Der von dem Mischstab 7 vermittelte Druck löst den Dorn 34 von der vorderen Wandung der Aufnahme 2 oder der Mischstab 7 durchstößt den Dorn 34. Gleichzeitig wird auch der Austragskolben 12 im Innenraum 11 in Richtung des Kartuschendeckels 6 getrieben.

Bei weiterem Einschrauben der Aufnahme 2 in die Kartusche 1 wird der Knochenzement 48 aus dem Innenraum 11 der Kartusche 1 durch die geöffnete Austragsöffnung ausgetrieben. Hierzu wird der Verschluss 38 zuvor aus der Austragsöffnung herausgeschraubt und ein Austragsrohr 49 wird in das Innengewinde des Stutzens 37 geschraubt. Das Austragsrohr 49 hat dafür ein zum Innengwinde des Stutzens 37 passendes Außengewinde. Der Knochenzement 48 wird zwischen dem Mischer 10 und dem Kartuschendeckel 6 durch die Austragsöffnung und den Stutzen 37 in das Austragsrohr 49 gepresst. Anschließend fließt der Knochenzement 48 aus dem Austragsrohr 49 hinaus und ist zur Anwendung bereit (siehe Figur 7).

Während des Auspressens des Knochenzements 48 werden Gaseinschlüsse in dem Knochenzement 48 durch den Porenfilter 16 nach oben in die Aufnahme 2 gedrückt, so dass ein blasenarmer Knochenzement 48 erzeugt wird.

Alternativ zu dem Austragsrohr 49 kann auch ein Schlauch mit einem Trokar (nicht gezeigt) an dem Stutzen 37 befestigt werden, durch die der Knochenzement 48 an schwer zugänglichen Orten unter Röntgenkontrolle appliziert werden kann.

### Bezugszeichenliste

- 1: Kartusche
- 2: Aufnahme / Ampullenhalter
- 3: Monomerflüssigkeit
- 4: Knochenzementpulver
- 5: Ampulle
- 6: Kartuschendeckel / Kartuschenkopf
- 7: Mischstab
- 8: Innengewinde der Kartusche
- 9: Gewinderohr
- 10: Mischer / Mischflügel
- 11: Innenraum der Kartusche
- 12: Austragskolben
- 13: Raum des Gewinderohrs
- 14: Kanal im Förderkolben
- 15: Innengewinde der Aufnahme
- 16: Porenfilter
- 18: Öffnungseinrichtung
- 20: Hülse
- 21: Schulter
- 22: Fixierungskappe
- 23: Hinteres Innengewinde der Fixierungskappe
- 24: Vorderes Innengewinde der Fixierungskappe
- 26: Außengewinde der Hülse
- 27: Ampullenboden
- 28: Griff
- 30: Dichtung
- 32: Dichtung
- 34: Dorn
- 36: Durchgang der Aufnahme
- 37: Stutzen
- 38: Verschluss
- 39: Flügel
- 40: Innengewinde des Kartuschendeckels
- 42: Außengewinde der Kartusche
- 43: Dichtung
- 45: Außengewinde des Gewinderohrs
- 47: Leiste
- 48: Knochenzement
- 49: Austragsrohr

## Patentansprüche

1. Knochenzementapplikator zum Lagern und Mischen eines Knochenzementpulvers (4) und einer Monomerflüssigkeit (3) sowie zum Applizieren eines aus dem Knochenzementpulver (4) und der Monomerflüssigkeit (3) gemischten pastösen Knochenzements (48), der Knochenzementapplikator aufweisend eine Kartusche (1) mit einem zylindrischen Innenraum (11) zum Mischen des Knochenzements (48), wobei die Kartusche (1) an einer Vorderseite einen Kartuschenkopf (6) mit einer Austragsöffnung zum Austreiben des Knochenzements (48) aus dem Innenraum (11) aufweist und wobei die Kartusche (1) an einer der Vorderseite der Kartusche (1) gegenüberliegenden Rückseite ein Gewinde (8) aufweist, einen Austragskolben (12) zum Austreiben des gemischten Knochenzements (48) aus dem Innenraum (11) durch die Austragsöffnung, wobei das Knochenzementpulver (4) zwischen dem Austragskolben (12) und dem Kartuschenkopf (6) in dem Innenraum (11) der Kartusche (1) enthalten ist,
ein Gewinderohr (9), das an der von dem Kartuschenkopf (6) abgewandten Rückseite des Austragskolbens (12) angeordnet ist, wobei das Gewinderohr (9) ein zu dem Gewinde (8) an der Rückseite der Kartusche (1) passendes Gegengewinde (45) aufweist und das Gegengewinde (45) des Gewinderohrs (9) in das Gewinde (8) an der Rückseite der Kartusche (1) greift, wobei sich das Gewinderohr (9) an der Rückseite der Kartusche (1) aus der Kartusche (1) heraus erstreckt und wobei der Austragskolben (12) durch Schrauben des Gewinderohrs (9) in oder auf die Kartusche (1) im Innenraum (11) der Kartusche (1) entlang der Zylinderachse des Innenraums (11) in Richtung des Kartuschenkopfs (6) beweglich angeordnet ist,
eine Aufnahme (2), wobei ein Monomerflüssigkeitsbehälter (5) im Inneren der Aufnahme (2) angeordnet ist, wobei der Monomerflüssigkeitsbehälter (5) die Monomerflüssigkeit (3) enthält und im Inneren der Aufnahme (2) zu öffnen ist, wobei die Aufnahme (2) an einer dem Austragskolben (12) gegenüberliegenden Rückseite des Gewinderohrs (9) in dem Gewinderohr (9) steckt und in dem Gewinderohr (9) beweglich ist, und
einen Mischstab (7), wobei der Mischstab (7) mit einem daran befestigten Mischer (10) in dem Innenraum (11) der Kartusche (1) angeordnet ist, wobei der Mischer (10) an einer dem Kartuschenkopf (6) zugewandten Vorderseite des Mischstabs (7) befestigt ist, wobei der Mischstab (7) an einer dem Mischer (10) gegenüberliegenden Seite mit einer dem Austragskolben (12) zugewandten Vorderseite der Aufnahme (2) verbunden ist, wobei der Mischstab (7) durch eine Durchführung in dem Austragskolben (12) geführt ist und in der Durchführung axial beweglich gelagert ist, so dass der Mischstab (7) mit dem Mischer (10) in dem Innenraum (11) zum Durchmischen des Knochenzementpulvers (4) mit der Monomerflüssigkeit (3) durch eine Bewegung der Aufnahme (2) gegen die Kartusche (1) beweglich ist, und wobei der Mischstab (7) lösbar mit der Aufnahme (2) verbunden ist und der von der Aufnahme (2) gelöste Mischstab (7) bei einem Vortreiben der Aufnahme (2) in Richtung des Kartuschenkopfs (6) in die Aufnahme (2) hinein drückbar ist.

2. Knochenzementapplikator nach Anspruch 1, **dadurch gekennzeichnet, dass** das Gewinde (8) an der Rückseite der Kartusche (1) ein Innengewinde (8) ist und das Gegengewinde (45) am Gewinderohr (9) ein Außengewinde (45) ist, so dass der Austragskolben (12) durch Einschrauben des Gewinderohrs (9) in den Innenraum (11) der Kartusche (1) im Innenraum (11) der Kartusche (1) axial entlang der Zylinderachse des Innenraums (11) in Richtung des Kartuschenkopfs (6) bewegbar ist.

3. Knochenzementapplikator nach Anspruch 1 oder 2, **dadurch gekennzeichnet, dass** das Gewinderohr (9) einen zylindrischen Raum (13) aufweist, der an der Rückseite des Gewinderohrs (9) offen ist, wobei die Aufnahme (2) von der Rückseite des Gewinderohrs (9) aus in dem zylindrischen Raum (13) des Gewinderohrs (9) steckt.

4. Knochenzementapplikator nach Anspruch 3, **dadurch gekennzeichnet, dass** die Aufnahme (2) gegen den zylindrischen Raum (13) des Gewinderohrs (9) abgedichtet ist, wobei die Aufnahme (2) in dem Gewinderohr (9) gleitend gelagert ist.

5. Knochenzementapplikator nach einem der Ansprüche 1 bis 3, **dadurch gekennzeichnet, dass**
das Gewinderohr (9) an seiner Rückseite eine zylindrische Durchführung aufweist, die gegen die Aufnahme (2) abgedichtet ist, wobei die Aufnahme (2) in der Durchführung gleitend gelagert ist.

6. Knochenzementapplikator nach einem der vorangehenden Ansprüche, **dadurch gekennzeichnet, dass**
der Mischstab (7) durch einen Druck auf den am Kartuschenkopf (6) anliegenden Mischer (10) und/oder durch ein Drehen oder Schrauben des Gewinderohrs (9) mit der Aufnahme (2) gegen den gegen eine Drehung im Innenraum (11) gesicherten Mischer (10) von der Aufnahme (2) lösbar ist.

7. Knochenzementapplikator nach einem der Ansprüche 4 oder 5, **dadurch gekennzeichnet, dass**
der Austragskolben (12) nicht durch eine Bewegung des Mischstabs (7) durch die Durchführung im Austragskolben (12) innerhalb des Innenraums (11) bewegbar ist.

8. Knochenzementapplikator nach einem der vorangehenden Ansprüche, **dadurch gekennzeichnet, dass**
ein Verbindungsmittel (22), insbesondere eine Fixierungskappe (22), vorgesehen ist, mit dem die Aufnahme (2) an dem Gewinderohr (9) fixierbar ist, wobei vorzugsweise die Fixierungskappe (22) über ein hinteres Innengewinde (23) mit einem kleineren Durchmesser als das Gewinde (45) des Gewinderohrs (9) mit einem Außengewinde einer Hülse (20) einer Öffnungseinrichtung (18) verbunden ist, wobei die Hülse (20) im Inneren der Aufnahme (2) auf den Monomerflüssigkeitsbehälter (5) drückbar ist, um den Monomerflüssigkeitsbehälter (5) zu öffnen, und wobei die Fixierungskappe (22) ein vorderes Innengewinde (24) aufweist, das zu dem Gewinde (45) des Gewinderohrs (9) passt.

9. Knochenzementapplikator nach einem der vorangehenden Ansprüche, **dadurch gekennzeichnet, dass**
an der Aufnahme (2) eine von außen bedienbare Öffnungseinrichtung (18) angeordnet ist, mit der der Monomerflüssigkeitsbehälter (5) im Inneren der Aufnahme (2) zu öffnen ist.

10. Knochenzementapplikator nach einem der vorangehenden Ansprüche, **dadurch gekennzeichnet, dass**
das Innere der Aufnahme (2) mit einem Innenraum (11) der Kartusche (1) flüssigkeitsdurchlässig verbunden ist, wobei bevorzugt hierzu die dem Kartuschenkopf (6) zugewandte Vorderseite der Aufnahme (2) wenigstens einen flüssigkeitsdurchlässigen Durchgang (36) und der Austragskolben (12) wenigstens einen flüssigkeitsdurchlässigen Kanal (14) aufweisen.

11. Knochenzementapplikator nach einem der vorangehenden Ansprüche, **dadurch gekennzeichnet, dass**
der Kartuschenkopf (6) ein auf die Kartusche (1) aufschraubbarer Kartuschendeckel (6) ist, wobei der Kartuschendeckel (6) den Innenraum (11) der Kartusche (1) an deren Vorderseite gasdicht und flüssigkeitsdicht abdichtet und wobei die Austragsöffnung in dem Kartuschendeckel (6) angeordnet ist, vorzugsweise in einem Stutzen (37) im Kartuschendeckel (6) angeordnet ist.

12. Knochenzementapplikator nach einem der vorangehenden Ansprüche, **dadurch gekennzeichnet, dass**
an der ins Innere der Aufnahme (2) weisenden Seite der Aufnahme (2) ein Dorn (34) zum Öffnen des Monomerflüssigkeitsbehälters (5) angeordnet ist.

13. Knochenzementapplikator nach einem der vorangehenden Ansprüche, **dadurch gekennzeichnet, dass**
der Mischstab (7) in seiner Verbindung zur Aufnahme (2) eine Kreisscheibe mit einem Außengewinde aufweist, wobei die Kreisscheibe in ein passendes Innengewinde an der dem Kartuschenkopf (6) zugewandten Vorderseite der Aufnahme (2) geschraubt ist, wobei bevorzugt das Außengewinde der Kreisscheibe und das Innengewinde an der Vorderseite der Aufnahme (2) Linksgewinde sind.

14. Verfahren zur Herstellung eines Knochenzements (48) mit einem Knochenzementapplikator nach einem der vorangehenden Ansprüche umfassend die folgenden Schritte
A) Öffnen des Monomerflüssigkeitsbehälters (5) im Inneren der Aufnahme (2) und Ausfließen der Monomerflüssigkeit (3) aus dem Monomerflüssigkeitsbehälter (5), wobei die Monomerflüssigkeit (3) aus der Aufnahme (2) in das Knochenzementpulver (4) im Innenraum (11) der Kartusche (1) fließt,
B) abwechselndes Herausziehen der Aufnahme (2) aus dem Gewinderohr (9) und Einschieben der Aufnahme (2) in das Gewinderohr (9), wobei der an der Vorderseite der Aufnahme (2) lösbar befestigte Mischstab (7) durch die Durchführung in dem Austragskolben (12) bewegt wird, wobei durch diese Bewegung der Mischer (10) in dem Innenraum (11) der Kartusche (1) bewegt wird und dadurch das Knochenzementpulver (4) und die Monomerflüssigkeit (3) miteinander zum Knochenzement (48) gemischt werden,
C) Lösen des Mischstabs (7) von der Aufnahme (2) durch Aufschrauben oder Einschrauben des Gewinderohrs (9) mit der Aufnahme (2) in den Innenraum (11) der Kartusche (1),
D) Öffnen der Austragsöffnung, und
E) Auspressen des Knochenzements (48) aus dem Innenraum (11) der Kartusche (1) durch die geöffnete Austragsöffnung, wobei der Knochenzement (48) mit dem Austragskolben (12) aus dem Innenraum (11) der Kartusche (1) gepresst wird und der Austragskolben (12) durch Aufschrauben des Gewinderohrs (9) auf die Kartusche (1) oder durch Einschrauben des Gewinderohrs (9) in die Kartusche (1) angetrieben wird und wobei beim Auf- oder Einschrauben des Gewinderohrs (9) der Mischstab (7) in die Aufnahme (2) hineingedrückt wird.

15. Verfahren nach Anspruch 14, **dadurch gekennzeichnet, dass**
der Austragskolben (12) wenigstens einen für das Knochenzementpulver (4) undurchlässigen und für die Monomerflüssigkeit (3) und Gase durchlässigen Kanal (14) aufweist, wobei
in Schritt A) die Monomerflüssigkeit (3) durch den Austragskolben (12) hindurch in den Innenraum (11) der Kartusche (1) fließt und in Schritt E) der Austragskolben (12) durch Einschrauben des Gewinderohrs (9) in die Kartusche (1) oder Aufschrauben des Gewinderohrs (9) auf die Kartusche (1) in Richtung des Kartuschenkopfs (6) gedrückt wird.

16. Verfahren nach Anspruch 15, **dadurch gekennzeichnet, dass**
ein in dem Knochenzement (48) enthaltenes Gas in Schritt E) beim Eindrücken oder Aufschrauben oder Einschrauben des Gewinderohrs (9) in den Innenraum (11) der Kartusche (1) durch den wenigstens einen Kanal (14) im Austragskolben (12) aus dem Knochenzement (48) herausgepresst wird.

17. Verfahren nach einem der Ansprüche 14 bis 16, **dadurch gekennzeichnet, dass**
der Monomerflüssigkeitsbehälter (5) in Schritt A) durch das Einschieben oder Einschrauben einer Öffnungseinrichtung (18) in die Aufnahme (2) geöffnet wird.

## Claims

1. Bone cement applicator for storage and mixing of a bone cement powder (4) and a monomer liquid (3) as well as for application of a pasty bone cement (48) produced by mixing the bone cement powder (4) and the monomer liquid (3), the bone cement applicator comprising
a cartridge (1) with a cylindrical internal space (11) for mixing the bone cement (48), whereby the cartridge (1) comprises, on a front side, a cartridge head (6) with a dispensing opening for expelling the bone cement (48) from the internal space (11), and whereby the cartridge (1) comprises a thread (8) on a rear side that is situated opposite from the front side of the cartridge (1);
a dispensing plunger (12) for expelling the mixed bone cement (48) from the internal space (11) through the dispensing opening, whereby the bone cement powder (4) is contained between the dispensing plunger (12) and the cartridge head (6) in the internal space (11) of the cartridge (1);
a threaded tube (9) that is arranged on the rear side of the dispensing plunger (12) that faces away from the cartridge head (6), whereby the threaded tube (9) comprises a counter-thread (45) that fits the thread (8) on the rear side of the cartridge (1), and the counter-thread (45) of the threaded tube (9) engages the thread (8) on the rear side of the cartridge (1), whereby the threaded tube (9) projects out of the cartridge (1) on the rear side of the cartridge (1), and whereby the dispensing plunger (12) is arranged such as to be mobile in the internal space (11) of the cartridge (1) along the cylinder axis of the internal space (11) in the direction of the cartridge head (6) by screwing the threaded tube (9) into or onto the cartridge (1);
a receptacle (2), whereby a monomer liquid container (5) is arranged on the inside of the receptacle (2), whereby the monomer liquid container (5) contains the monomer liquid (3) and can be opened on the inside of the receptacle (2), whereby the receptacle (2) is plugged into the threaded tube (9) on a rear side of the threaded tube (9) that is situated opposite from the dispensing plunger (12), and is mobile in the threaded tube (9); and
a mixing rod (7), whereby the mixing rod (7) with a mixer (10) affixed to it is arranged in the internal space (11) of the cartridge (1), whereby the mixer (10) is affixed on a front side of the mixing rod (7) that faces the cartridge head (6), whereby the mixing rod (7) is connected, on a side that is situated opposite from the mixer (10), to a front side of the receptacle (2) that faces the dispensing plunger (12), whereby the mixing rod (7) is conducted through a feedthrough in the dispensing plunger (12) and is supported in the feedthrough such as to be axially mobile, such that the mixing rod (7) with the mixer (10) is mobile in the internal space (11) for the mixing of the bone cement powder (4) with the monomer liquid (3) through a motion of the receptacle (2) against the cartridge (1), and whereby the mixing rod (7) is detachably connected to the receptacle (2), and the mixing rod (7), detached from the receptacle (2), can be pushed into the receptacle (2) upon the receptacle (2) being propelled in the direction of the cartridge head (6).

2. Bone cement applicator according to claim 1, **characterised in that** the thread (8) on the rear side of the cartridge (1) is an internal thread (8) and the counter-thread (45) on the threaded tube (9) is an external thread (45) such that the dispensing plunger (12) can be moved in the internal space (11) of the cartridge (1) along the cylinder axis of the internal space (11) in the direction of the cartridge head (6) by screwing the threaded tube (9) into the internal space (11) of the cartridge (1).

3. Bone cement applicator according to claim 1 or 2, **characterised in that** the threaded tube (9) comprises a cylindrical space (13) that is open on the rear side of the threaded tube (9), whereby the receptacle (2) is plugged, originating from the rear side of the threaded tube (9), into the cylindrical space (13) of the threaded tube (9).

4. Bone cement applicator according to claim 3, **characterised in that** the receptacle (2) is sealed with respect to the cylindrical space (13) of the threaded tube (9), whereby the receptacle (2) is supported in the threaded tube (9) in a gliding manner.

5. Bone cement applicator according to any one of the claims 1 to 3, **characterised in that**
the threaded tube (9), on its rear side, comprises a cylindrical feedthrough that is sealed with respect to the receptacle (2), whereby the receptacle (2) is supported in the feedthrough in a gliding manner.

6. Bone cement applicator according to any one of the preceding claims,
**characterised in that**
the mixing rod (7) can be detached from the receptacle (2) by pushing onto the mixer (10) touching against the cartridge head (6) and/or by rotating or screwing the threaded tube (9) with the receptacle (2) with respect to the mixer (10) that is secured against a rotation in the internal space (11).

7. Bone cement applicator according to any one of the claims 4 or 5,
**characterised in that**
the dispensing plunger (12) cannot be moved within the internal space (11) through a motion of the mixing rod (7) through the feedthrough in the dispensing plunger (12).

8. Bone cement applicator according to any one of the preceding claims,
**characterised in that**
a connecting means (22), in particular a fixation cap (22), is provided by means of which the receptacle (2) can be affixed to the threaded tube (9), whereby the fixation cap (22) preferably is connected by means of a rear internal thread (23) with a smaller diameter than the thread (45) of the threaded tube (9) to an external thread of a sleeve (20) of an opening facility (18), whereby the sleeve (20) can be pushed onto the monomer liquid container (5) on the inside of the receptacle (2) in order to open the monomer liquid container (5), and whereby the fixation cap (22) comprises a front internal thread (24) that fits the thread (45) of the threaded tube (9).

9. Bone cement applicator according to any one of the preceding claims,
**characterised in that**
an opening facility (18), which can be operated from outside and by means of which the monomer liquid container (5) can be opened on the inside of the receptacle (2), is arranged on the receptacle (2).

10. Bone cement applicator according to any one of the preceding claims,
**characterised in that**
the inside of the receptacle (2) is connected in liquid-permeable manner to an internal space (11) of the cartridge (1), whereby, for this purpose, the front side of the receptacle (2) facing the cartridge head (6) preferably comprises at least one liquid-permeable passage (36), and the dispensing plunger (12) comprises at least one liquid-permeable channel (14).

11. Bone cement applicator according to any one of the preceding claims,
**characterised in that**
the cartridge head (6) is a cartridge lid (6) that can be screwed onto the cartridge (1), whereby the cartridge lid (6) seals the internal space (11) of the cartridge (1) on the front side thereof in gas-tight and liquid-tight manner, and whereby the dispensing opening is arranged in the cartridge lid (6), preferably is arranged in a socket (37) in the cartridge lid (6).

12. Bone cement applicator according to any one of the preceding claims,
**characterised in that**
a mandrel (34) for opening of the monomer liquid container (5) is arranged on the side of the receptacle (2) pointing into the inside of the receptacle (2).

13. Bone cement applicator according to any one of the preceding claims,
**characterised in that**
the mixing rod (7) comprises, in its connection to the receptacle (2), a circular disk with an external thread, whereby the circular disk is screwed into a fitting internal thread on the front side of the receptacle (2) facing the cartridge head (6), whereby the external thread of the circular disk and the internal thread on the front side of the receptacle (2) are preferred to be left-hand threads.

14. Method for producing a bone cement (48) with a bone cement applicator according to any one of the preceding claims, comprising the following steps of
A) Opening the monomer liquid container (5) on the inside of the receptacle (2) and the monomer liquid (3) flowing from the monomer liquid container (5), whereby the monomer liquid (3) flows from the receptacle (2) into the bone cement powder (4) in the internal space (11) of the cartridge (1);
B) alternatingly pulling the receptacle (2) from the threaded tube (9) and inserting the receptacle (2) into the threaded tube (9), whereby the mixing rod (7), which is detachably affixed to the front side of the receptacle (2), is being moved through the feedthrough in the dispensing plunger (12), whereby said motion moves the mixer (10) in the internal space (11) of the cartridge (1) and thus mixes the bone cement powder (4) and the monomer liquid (3) with each other to form bone cement (48);
C) detaching the mixing rod (7) from the receptacle (2) by screwing the threaded tube (9) with the receptacle (2) onto or into the internal space (11) of the cartridge (1);
D) opening the dispensing opening; and
E) extruding the bone cement (48) from the internal space (11) of the cartridge (1) through the opened dispensing opening, whereby the bone cement (48) is extruded from the internal space (11) of the cartridge (1) by means of the dispensing plunger (12), and the dispensing plunger (12) is driven by screwing the threaded tube (9) onto the cartridge (1) or by screwing the threaded tube (9) into the cartridge (1), and whereby the mixing rod (7) is being pushed into the receptacle (2) while the threaded tube (9) is being screwed on or in.

15. Method according to claim 14, **characterised in that**
the dispensing plunger (12) comprises at least one channel (14) that is impermeable to the bone cement powder (4) and is permeable to the monomer liquid (3) and gases, whereby
in step A), the monomer liquid (3) flows through the dispensing plunger (12) into the internal space (11) of the cartridge (1) and, in step E), the dispensing plunger (12) is being pushed in the direction of the cartridge head (6) by screwing the threaded tube (9) into the cartridge (1) or by screwing the threaded tube (9) onto the cartridge (1).

16. Method according to claim 15, **characterised in that** a gas contained in the bone cement (48) is squeezed out from the bone cement (48) in step E) through the at least one channel (14) in the dispensing plunger (12) while the threaded tube (9) is being pushed into or screwed onto or screwed into the internal space (11) of the cartridge (1).

17. Method according to any one of the claims 14 to 16, **characterised in that** the monomer liquid container (5) is being opened in step A) by inserting or screwing an opening facility (18) into the receptacle (2).

## Revendications

1. Applicateur de ciment osseux pour le stockage et le mélange d'une poudre de ciment osseux (4) et d'un liquide monomère (3) ainsi que pour l'application d'un ciment osseux (48) pâteux, mélangé à partir de la poudre de ciment osseux (4) et du liquide monomère (3), l'applicateur de ciment osseux présentant
une cartouche (1) avec un espace intérieur (11) cylindrique pour le mélange du ciment osseux (48), la cartouche (1) présentant, sur une face avant, une tête de cartouche (6) avec un orifice de sortie pour l'éjection du ciment osseux (48) de l'espace intérieur (11) et la cartouche (1) présentant, sur une face arrière opposée à la face avant de la cartouche (1), un filetage (8),
un piston de décharge (12) pour l'éjection du ciment osseux (48) mélangé de l'espace intérieur (11) par l'orifice de sortie, la poudre de ciment osseux (4) étant contenue entre le piston de décharge (12) et la tête de cartouche (6) dans l'espace intérieur (11) de la cartouche (1),
un tube fileté (9) qui est disposé sur la face arrière du piston de décharge (12), détournée de la tête de cartouche (6), le tube fileté (9) présentant un contre-filetage (45) adapté au filetage (8) sur la face arrière de la cartouche (1), et le contre-filetage (45) du tube fileté (9) entrant dans le filetage (8) sur la face arrière de la cartouche (1), le tube fileté (9) s'étendant depuis la cartouche (1) sur la face arrière de la cartouche (1) et le piston de décharge (12) étant déplaçable le long de l'axe cylindrique de l'espace intérieur (11) en direction de la tête de cartouche (6) par le vissage du tube fileté (9) dans ou sur la cartouche (1) dans l'espace intérieur (11) de la cartouche (1),
un logement (2), un réservoir de liquide monomère (5) étant disposé à l'intérieur du logement (2), le réservoir de liquide monomère (5) contenant le liquide monomère (3) et pouvant être ouvert à l'intérieur du logement (2), le logement (2) s'insérant dans le tube fileté (9) sur une face arrière du tube fileté (9), opposée au piston de décharge (12) et étant déplaçable dans le tube fileté (9), et
une barre de mélange (7), la barre de mélange (7) étant disposée avec un mélangeur (10) fixé sur celle-ci dans l'espace intérieur (11) de la cartouche (1), le mélangeur (10) étant fixé sur une face avant de la barre de mélange (7), tournée vers la tête de cartouche (6), la barre de mélange (7) étant reliée, sur une face opposée au mélangeur (10), à une face avant du logement (2) tournée vers le piston de décharge (12), la barre de mélange (7) étant guidée à travers un passage dans le piston de décharge (12) et étant déplaçable axialement dans le passage de façon à ce que la barre de mélange (7) soit déplaçable avec le mélangeur (10) dans l'espace intérieur (11) pour le mélange de la poudre de ciment osseux (4) avec le liquide monomère (3) par un déplacement du logement (2) contre la cartouche (1), la barre de mélange (7) étant reliée de manière amovible au logement (2) et la barre de mélange (7) retirée du logement (2) pouvant être enfoncée dans le logement (2) lors d'un avancement du logement (2) en direction de la tête de cartouche (6).

2. Applicateur de ciment osseux conformément à la revendication n°1,
**caractérisé en ce que**
le filetage (8) sur la face arrière de la cartouche (1) est un filetage intérieur (8) et le contre-filetage (45) sur le tube fileté (9) est un filetage extérieur (45) de façon à ce que le piston de décharge (12) soit déplaçable axialement le long de l'axe cylindrique de l'espace intérieur (11) en direction de la tête de cartouche (6) par le vissage du tube fileté (9) dans l'espace intérieur (11) de la cartouche (1).

3. Applicateur de ciment osseux conformément à la revendication n°1 ou n°2,
**caractérisé en ce que**
le tube fileté (9) présente un espace cylindrique (13) qui est ouvert sur la face arrière du tube fileté (9), le logement (2) s'insérant dans l'espace cylindrique (13) du tube fileté (9) depuis la face arrière du tube fileté (9).

4. Applicateur de ciment osseux conformément à la revendication n°3,
**caractérisé en ce que**
le logement (2) est étanchéifié contre l'espace cylindrique (13) du tube fileté (9), le logement (2) étant monté sur palier coulissant dans le tube fileté (9).

5. Applicateur de ciment osseux conformément à l'une des revendications n°1 à n°3, **caractérisé en ce que**
le tube fileté (9) présente, sur sa face arrière, un passage cylindrique qui est étanchéifié contre le logement (2), le logement (2) étant monté sur palier coulissant dans le passage.

6. Applicateur de ciment osseux conformément à l'une des revendications précédentes, **caractérisé en ce que**
la barre de mélange (7) peut être retirée du logement (2) par une pression sur le mélangeur (10) adjacent à la tête de cartouche (6) et/ou par une rotation ou un vissage du tube fileté (9) avec le logement (2) contre le mélangeur (10) sécurisé contre une rotation dans l'espace intérieur (11).

7. Applicateur de ciment osseux conformément à l'une des revendications n°4 ou n°5, **caractérisé en ce que**
le piston de décharge (12) n'est pas déplaçable par un déplacement de la barre de mélange (7) à travers le passage dans le piston de décharge (12) à l'intérieur de l'espace intérieur (11).

8. Applicateur de ciment osseux conformément à l'une des revendications précédentes, **caractérisé en ce que**
un moyen de liaison (22), en particulier un bonnet de fixation (22), avec lequel le logement (2) peut être fixé sur le tube fileté (9), est prévu, le bonnet de fixation (22) étant préférablement relié à un filetage extérieur d'un manchon (20) d'un dispositif d'ouverture (18) par un filetage intérieur (23) arrière avec un plus petit diamètre que le filetage (45) du tube fileté (9), le manchon (20) pouvant être enfoncé à l'intérieur du logement (2) sur le réservoir de liquide monomère (5) pour ouvrir le réservoir de liquide monomère (5), et le bonnet de fixation (22) présentant un filetage intérieur (24) frontal qui est adapté au filetage (45) du tube fileté (9).

9. Applicateur de ciment osseux conformément à l'une des revendications précédentes, **caractérisé en ce que**
un dispositif d'ouverture (18) pouvant être utilisé depuis l'extérieur, avec lequel le réservoir de liquide monomère (5) peut être ouvert à l'intérieur du logement (2), est disposé sur le logement (2).

10. Applicateur de ciment osseux conformément à l'une des revendications précédentes, **caractérisé en ce que**
l'intérieur du logement (2) est relié, en étant perméable aux liquides, à un espace intérieur (11) de la cartouche (1), la face avant du logement (2), tournée vers la tête de cartouche (6), présentant préférablement au moins un passage (36) perméable aux liquides et le piston de décharge (12) présentant au moins un conduit (14) perméable aux liquides.

11. Applicateur de ciment osseux conformément à l'une des revendications précédentes, **caractérisé en ce que**
la tête de cartouche (6) est un couvercle de cartouche (6) dévissable sur la cartouche (1), le couvercle de cartouche (6) étanchéifiant hermétiquement aux gaz et aux liquides l'espace intérieur (11) de la cartouche (1) sur sa face avant et l'orifice de sortie étant disposé dans le couvercle de cartouche (6), préférablement dans un embout (37) dans le couvercle de cartouche (6).

12. Applicateur de ciment osseux conformément à l'une des revendications précédentes, **caractérisé en ce que**
un mandrin (34) pour l'ouverture du réservoir de liquide monomère (5) est disposé sur la face du logement (2), tournée vers l'intérieur du logement (2).

13. Applicateur de ciment osseux conformément à l'une des revendications précédentes, **caractérisé en ce que**
la barre de mélange (7) présente, dans sa liaison avec le logement (2), un disque avec un filetage extérieur, le disque étant vissé dans un filetage intérieur adéquat sur la face avant du logement (2), tournée vers la tête de cartouche (6), le filetage extérieur du disque et le filetage intérieur sur la face avant du logement (2) étant préférablement des filetages à gauche.

14. Procédé de fabrication d'un ciment osseux (48) avec un applicateur de ciment osseux conformément à l'une des revendications précédentes, comprenant les étapes suivantes :
A) Ouverture du réservoir de liquide monomère (5) à l'intérieur du logement (2) et écoulement du liquide monomère (3) du réservoir de liquide monomère (5), le liquide monomère (3) s'écoulant du logement (2) dans la poudre de ciment osseux (4) dans l'espace intérieur (11) de la cartouche (1)
B) En alternance, retrait du logement (2) du tube fileté (9) et insertion du logement (2) dans le tube fileté (9), la barre de mélange (7) fixée de façon amovible sur la face avant du logement (2) étant déplacée à travers le passage dans le piston de décharge (12), le mélangeur (10) étant déplacé dans l'espace intérieur (11) de la cartouche (1) par ce déplacement et la poudre de ciment osseux (4) et le liquide monomère (3) étant ainsi mélangés en un ciment osseux (48)
C) Retrait de la barre de mélange (7) du logement (2) par le dévissage ou le vissage du tube fileté (9) avec le logement (2) dans l'espace intérieur (11) de la cartouche (1)
D) Ouverture de l'orifice de sortie et
E) Pressage du ciment osseux (48) de l'espace intérieur (11) de la cartouche (1) par l'orifice de sortie ouvert, le ciment osseux (48) étant pressé de l'espace intérieur (11) de la cartouche (1) avec le piston de décharge (12) et le piston de décharge (12) étant entraîné par le dévissage du tube fileté (9) sur la cartouche (1) ou par le vissage du tube fileté (9) dans la cartouche (1) et la barre de mélange (7) étant enfoncée dans le logement (2) lors du dévissage ou du vissage du tube fileté (9).

15. Procédé conformément à la revendication n°14, **caractérisé en ce que**
le piston de décharge (12) présente au moins un conduit (14) imperméable à la poudre de ciment osseux (4) et perméable au liquide monomère (3) et aux gaz,
dans l'étape A), le liquide monomère (3) coulant dans l'espace intérieur (11) de la cartouche (1) en passant par le piston de décharge (12) et, dans l'étape E), le piston de décharge (12) étant enfoncé en direction de la tête de cartouche (6) par le vissage du tube fileté (9) dans la cartouche (1) ou par le dévissage du tube fileté (9) sur la cartouche (1).

16. Procédé conformément à la revendication n°15, **caractérisé en ce que**
un gaz contenu dans le ciment osseux (48) est pressé du ciment osseux (48) dans l'étape E) lors de l'insertion ou du dévissage ou du vissage du tube fileté (9) dans l'espace intérieur (11) de la cartouche (1) en passant par au moins un conduit (14) dans le piston de décharge (12).

17. Procédé conformément à l'une des revendications n°14 à n°16, **caractérisé en ce que**
le réservoir de liquide monomère (5) est ouvert dans l'étape A) par l'insertion ou le vissage d'un dispositif d'ouverture (18) dans le logement (2).
